(19) Europäisches Patentamt
European Patent Office
Office européen des brevets

(11) Veröffentlichungsnummer: **0 537 696 A1**

(12) # EUROPÄISCHE PATENTANMELDUNG

(21) Anmeldenummer: **92117507.1**

(22) Anmeldetag: **14.10.92**

(51) Int. Cl.5: **C07D 237/24**, C07D 239/36, C07D 213/64, C07D 237/14, C07D 237/20, C07D 401/06, C07F 9/6512, A61K 31/50

(30) Priorität: **18.10.91 DE 4134467**

(43) Veröffentlichungstag der Anmeldung: **21.04.93 Patentblatt 93/16**

(84) Benannte Vertragsstaaten: **AT BE CH DE DK ES FR GB GR IE IT LI LU NL PT SE**

(71) Anmelder: **Dr. Karl Thomae GmbH**
**Postfach 1755**
**W-7950 Biberach 1(DE)**

(72) Erfinder: **Linz, Günter, Dr. Dipl.-Chem.**
**Erlenweg 8**
**W-7951 Mittelbiberach(DE)**
Erfinder: **Pieper, Helmut, Dr. Dipl.-Chem.**
**Kapellenweg 5**
**W-7950 Biberach 1(DE)**
Erfinder: **Himmelsbach, Frank, Dr. Dipl.-Chem.**
**Ahornweg 16**
**W-7951 Mittelbiberach(DE)**
Erfinder: **Austel, Volkhard, Dr. Dipl.-Chem.**
**Kapellenweg 7**
**W-7950 Biberach 1(DE)**
Erfinder: **Müller, Thomas Dr. Arzt und Dipl.-Chem**
**Gymnasiumstrasse 16**
**W-7950 Biberach 1(DE)**
Erfinder: **Weisenberger, Johannes, Dr. Dipl.-Chem.**
**Haydnweg 5**
**W-7950 Biberach 1(DE)**
Erfinder: **Seewaldt-Becker, Elke, Dr. Dipl.-Bio.**
**Hühnerfeldstrasse 26**
**W-7950 Biberach(DE)**

(54) **Heterobiarylderivate, diese Verbindungen enthaltende Arzneimittel und Verfahren zu ihrer Herstellung.**

(57) Die Erfindung betrifft Heterobiarylderivate der allgemeinen Formel

$$R_1NH - X_1 - X_2 - X_3 - Y_1 - Y_2 - Y_3 - Y_4 - E \quad ,(I)$$

in der
$R_1$, $X_1$ bis $X_3$ und $Y_1$ bis $Y_4$ wie im Anspruch 1 definiert sind, deren Tautomere, deren Stereoisomere, deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel und Verfahren zu ihrer Herstellung.

EP 0 537 696 A1

Die Erfindung betrifft Heterobiarylderivate der allgemeinen Formel

$R_1NH - X_1 - X_2 - X_3 - Y_1 - Y_2 - Y_3 - Y_4 - E$ ,(I)

mit Ausnahme von 2-Guanidino-4-[3-[3-(methoxycarbonyl)-propyl]-phenyl]-1,3-thiazol (siehe C.A. 103, 71307m (1985)) und 2-Guanidino-4-[6-[(methoxycarbonyl)-methyl]-pyrid-2-yl]-1,3-thiazol (siehe Ep-A-0,417,751), welche Histamin-Rezeptor-Antagonisten darstellen,
deren Tautomere, deren Stereoisomere, einschließlich ihrer Gemische und deren Salze, insbesondere deren physiologisch verträgliche Salze mit anorganischen oder organischen Säuren oder Basen, welche u.a. wertvolle pharmakologische Eigenschaften aufweisen, vorzugsweise aggregationshemmende Wirkungen, diese Verbindungen enthaltende Arzneimittel und Verfahren zu ihrer Herstellung.

In der obigen allgemeinen Formel I bedeutet

$R_1$ ein Wasserstoffatom, eine Alkyl-, Hydroxy-, Amino-, Alkoxycarbonyl-, Aralkoxycarbonyl-, Aryloxycarbonyl-, Alkylcarbonyl- oder Arylcarbonylgruppe, in denen der Alkyl-und Alkoxyteil jeweils 1 bis 4 Kohlenstoffatome enthalten kann, eine Alkenyloxycarbonylgruppe mit insgesamt 4 bis 6 Kohlenstoffatomen, eine Phosphono-, O-Alkyl-phosphono-, Dialkylphosphoryl- oder R'-CO-O-(R''CH)-O-CO-Gruppe, wobei

R' eine Alkylgruppe mit 1 bis 5 Kohlenstoffatomen, eine Cycloalkyl-, Cycloalkylalkyl-, Aryl- oder Aralkylgruppe und

R'' ein Wasserstoffatom, eine Alkylgruppe mit 1 bis 5 Kohlenstoffatomen, eine Cycloalkyl- oder Arylalkylgruppe darstellen,

$X_1$ eine Alkylengruppe mit 1 bis 3 Kohlenstoffatomen, eine -C(=NH)-, -C(=NH)-NH- oder -C(=NH)-NH-CO-Gruppe, wobei jeweils das Kohlenstoffatom der -C(=NH)-Gruppe der vorstehend erwähnten Reste mit dem Stickstoffatom des $R_1NH$-Restes verknüpft ist,

$X_2$ eine Phenylen-, Pyridinylen-, Pyrazinylen-, Pyrimidinylen- oder Pyridazinylengruppe, die jeweils im Kohlenstoffgerüst durch Fluor-, Chlor-, Brom- oder Jodatome, durch Alkyl-, Aralkyl-, Aryl-, Pyridylalkyl-, Hydroxy-, Alkoxy-, Aralkoxy-, Pyridylalkoxy-, Alkylsulfenyl-, Alkylsulfinyl-, Alkylsulfonyl-, Amino-, Alkylamino-, Dialkylamino-, Alkylcarbonylamino-, Arylcarbonylamino-, Alkylsulfonylamino-, Arylsulfonylamino-, Aralkylamino-, Aminocarbonylalkyl-, Alkylaminocarbonylalkyl-, Dialkylaminocarbonylalkyl-, $R_2$-, $R_2CO$-alkyl- oder $R_3CO-CHR_4-(CH_2)_1$-NHCO-alkyl-Gruppen mono- oder oder disubstituiert sein können, wobei die Substituenten gleich oder verschieden sein können und zusätzlich in einem der vorstehend erwähnten ein oder zwei Stickstoffatome enthaltenden 6-gliedrigen heteroaromatischen Reste eine oder zwei -N=CH-Gruppen durch eine oder zwei -$NR_5$-CO-Gruppen ersetzt sein können, in denen

1 die Zahl 0 oder 1,

$R_2$ eine Azetidino-, Pyrrolidino-, Piperidino- oder Hexamethyleniminogruppe, wobei die Methylengruppe in 4-Stellung einer Piperidinogruppe zusätzlich durch eine -O-, -S-, -SO-, -$SO_2$-, -NH-, -N(Alkyl)-, -N(CHO)-, -N(COAlkyl)- oder -N(COAryl)-gruppe ersetzt sein kann,

$R_3$ eine Hydroxygruppe oder eine Alkoxygruppe mit 1 bis 5 Kohlenstoffatomen, in der der Alkoxyteil in 1-, 2-oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino- oder Thiomorpholinogruppe substituiert sein kann,

$R_4$ ein Wasserstoffatom, eine geradkettige oder verzweigte Alkylgruppe mit 1 bis 6 Kohlenstoffatomen, die durch eine Hydroxy-, Mercapto-, Alkylmercapto-, Amino-, $R_3CO$-, Aminocarbonyl-, Phenyl-, Indolyl- oder Imidazolylgruppe substituiert sein kann, wobei $R_3$ wie eingangs definiert ist und der Phenylrest durch ein Fluor-, Chlor-oder Bromatom, durch eine Hydroxy- oder Aminogruppe substituiert sein kann, und

$R_5$ ein Wasserstoffatom, eine Alkyl-, Aralkyl-, Aryl-, Pyridylalkyl-, Aminocarbonylalkyl-, Alkylaminocarbonyl alkyl-, Dialkylaminocarbonylalkyl-, $R_2CO$-alkyl- oder $R_3CO-CHR_4-(CH_2)_l$-NHCO-alkyl-Gruppe, wobei l, $R_2$, $R_3$ und $R_4$ wie vorstehend erwähnt definiert sind, darstellen,

eine Thiophenylen-, Thiazolylen- oder Thiadiazolylengruppe,

$X_3$ die für $X_2$ vorstehend erwähnten Bedeutungen mit der Maßgabe besitzt, daß $X_3$ keine Thiophenylen-, Thiazolylen- oder Thiadiazolylengruppe und mindestens einer der Reste $X_2$ oder $X_3$ einen der vorstehend erwähnten heteroaromatischen Reste darstellt, und $X_3$ eine 1,2,4-Triazinylengruppe, die im Kohlenstoffgerüst durch ein Fluor-, Chlor-, Brom- oder Jodatom, durch eine Alkyl-, Aralkyl-, Aryl-, Pyridylalkyl-, Hydroxy-, Alkoxy-, Aralkoxy-, Pyridylalkoxy-, Alkylsulfenyl-, Alkylsulfinyl-, Alkylsulfonyl-, Amino-, Alkylamino-, Dialkylamino-, Alkylcarbonylamino-, Arylcarbonylamino-, Alkylsulfonylamino-, Arylsulfonylamino-, Aralkylamino-, Aminocarbonylalkyl-, Alkylaminocarbonylalkyl-, Dialkylaminocarbonylalkyl-, $R_2$-, $R_2CO$-alkyl- oder $R_3CO-CHR_4-(CH_2)_l$-NHCO-alkyl-Gruppe substituiert sein kann, wobei l, $R_2$, $R_3$ und $R_4$ wie vorstehend erwähnt definiert sind und gleichzeitig in einer vorstehend erwähnten 1,2,4-Triazinylengruppe eine oder zwei -N=CH-Gruppen durch eine oder zwei -$NR_5$-CO-Gruppe ersetzt sein können, wobei $R_5$ wie vorste-

hend erwähnt definiert ist und zusätzlich in einem bei der Definition des Restes $X_3$ vorstehend erwähnten Ringe, die eine $-NR_5-CO-$Gruppe enthalten, $R_5$ eine Bindung zu dem Rest $X_2$ oder auch, wenn $Y_1$ eine Bindung darstellt, zu dem Rest $Y_2$ bedeuten kann,

$Y_1$ eine Bindung, eine -O-, -S-, -SO-, $-SO_2-$, -CO-, $-NR_6-$, $-NR_6CO-$, $-CONR_6-$, $-SO_2NR_6-$ oder $-NR_6SO_2-$Gruppe, wobei

$R_6$ ein Wasserstoffatom, eine Alkylgruppe mit 1 bis 3 Kohlenstoffatomen oder eine Aralkylgruppe darstellt,

oder auch eine $-OCH_2CO-$Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylen- oder Alkinylengruppe mit jeweils 2 bis 6 Kohlenstoffatomen, wobei die Doppelbindung nicht mit einem Sauerstoff-, Schwefel- oder Phosphoratom der Reste $Y_1$, $Y_3$ oder E und die Dreifachbindung nicht mit einem Heteroatom der Reste $Y_1$, $Y_3$ oder E direkt verknüpft sein kann, eine Cycloalkylengruppe mit 3 bis 7 Kohlenstoffatomen oder eine gegebenenfalls durch Fluor-, Chlor-, Brom- oder Jodatome, durch Alkyl-, Hydroxy-, Alkoxy-, Alkylsulfenyl-, Alkylsulfinyl-, Alkylsulfonyl-, Amino-, Alkylcarbonylamino- oder Alkylsulfonylaminogruppen mono- oder disubstituierte Arylengruppe, wobei die Substituenten gleich oder verschieden sein können,

$Y_3$ eine Bindung, eine -CO- oder $-CONR_6-$Gruppe oder auch, wenn ein Heteroatom des Restes $Y_1$ nicht an dasselbe Kohlenstoffatom des Restes $Y_2$ gebunden ist wie der Rest $Y_3$, eine -O-, -S-, -SO-, $-SO_2-$, $-NR_6CO-$ oder $-NR_7-$Gruppe, wobei

$R_7$ ein Wasserstoffatom, eine Alkyl-, Aralkyl-, Formyl-, Alkylcarbonyl-, Arylcarbonyl-, Aralkylcarbonyl-, Alkylsulfonyl-, Arylsulfonyl- oder Aralkylsulfonylgruppe darstellt,

eine Gruppe der Formeln

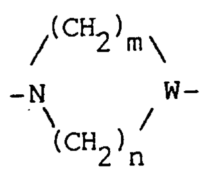

oder

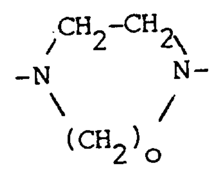

in denen

W eine >CH-, >C(OH)-, >CH-O-, >C=CH- oder $>C(CH_2COR_3)$-Gruppe, wobei $R_3$ wie vorstehend erwähnt definiert ist,

m und n jeweils die Zahlen 1, 2 oder 3, wobei jedoch m + n die Zahl 2, 3, 4, 5 oder 6 darstellen muß, und o die Zahl 2 oder 3 darstellen, wobei in den vorstehend erwähnten Ringen zusätzlich eine Methylengruppe durch eine Carbonylgruppe ersetzt sein kann oder auch, falls die Methylengruppe nicht benachbart zu einem Stickstoffatom steht, durch eine Hydroxygruppe substituiert sein kann, sowie

generell ein Sauerstoff- oder Schwefelatom des Restes $Y_3$ nicht unmittelbar auf ein Sauerstoff- oder Schwefelatom oder eine CO-Gruppe des Restes $Y_1$ und ein Sauerstoffatom oder eine Sulfenyl- oder Sulfinylgruppe des Restes $Y_3$ nicht unmittelbar auf ein Stickstoffatom des Restes $Y_1$ und eine CO-Gruppe des Restes $Y_3$ nicht unmittelbar auf eine -O-, -S-, -SO- oder $-SO_2-$Gruppe des Restes $Y_1$ folgen kann,

$Y_4$ eine Bindung, eine geradkettige oder verzweigte Alkylengruppe mit 1 bis 4 Kohlenstoffatomen oder eine gegebenenfalls durch Fluor-, Chlor-, Brom- oder Jodatome, durch Alkyl-, Hydroxy-, Alkoxy-, Alkylsulfenyl-, Alkylsulfinyl-, Alkylsulfonyl-, Amino-, Alkylcarbonylamino- oder Alkylsulfonylaminogruppen mono- oder disubstituierte Arylengruppe, wobei die Substituenten gleich oder verschieden sein können, und

E eine Sulfo-, 5-Tetrazolyl-, Phosphono-, O-Alkyl-phosphono-, Dialkylphosphoryl-, R'-CO-O-(R''CH)-O-CO-, R'''CO- oder R'O-CO-O-(R''CH)-O-CO-Gruppe, wobei

R' und R'' wie vorstehend erwähnt definiert sind und

R''' eine Hydroxygruppe, eine Alkoxygruppe mit 1 bis 5 Kohlenstoffatomen, in der der Alkoxyteil in 1-, 2- oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino- oder Thiomorpholinogruppe substituiert sein kann, eine Arylalkenyloxygruppe mit 3 oder 4 Kohlenstoffatomen im Alkenylteil, eine Cycloalkoxy- oder Cycloalkylalkoxygruppe darstellt,

und mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,

wobei, soweit nichts anderes erwähnt wurde,

unter "eine Aryl- oder Arylengruppe" eine gegebenenfalls durch Fluor-, Chlor-, Brom- oder Jodatome, durch

Alkyl-, Trifluormethyl-, Nitro-, Amino-, Alkylamino-, Dialkylamino-, Alkanoylamino-, Alkylsulfonylamino-, Aminosulfonyl-, Alkylaminosulfonyl-, Dialkylaminosulfonyl-, Hydroxy-, Alkoxy-, Carboxy-, Alkoxycarbonyl-, Aminocarbonyl-, Alkylaminocarbonyl-, Dialkylaminocarbonyl-, Alkylsulfenyl-, Alkylsulfinyl- oder Alkylsulfonylgruppen mono-, di- oder trisubstituierte Phenyl- oder Phenylengruppe, wobei die Substituenten gleich oder verschieden sein können, und

die vorstehend erwähnten Alkyl-, Alkylen- oder Alkoxyteile jeweils 1 bis 3 Kohlenstoffatome, die Cycloalkyl- und Cycloalkoxyteile jeweils 3 bis 7 Kohlenstoffatome und die Alkanoylteile 1 bis 4 Kohlenstoffatome enthalten können.

Bevorzugte Verbindungen der obigen allgemeinen Formel I sind jedoch diejenigen, in denen

$R_1$ ein Wasserstoffatom, eine Alkylgruppe mit 1 bis 4 Kohlenstoffatomen oder eine gegebenenfalls im Alkoxyteil durch eine Phenylgruppe substituierte Alkoxycarbonylgruppe mit insgesamt 2 bis 5 Kohlenstoffatomen, eine Phosphonogruppe, eine O-Alkyl-phosphono- oder Dialkylphosphorylgruppe, in denen der Alkylteil jeweils 1 oder 2 Kohlenstoffatome enthalten kann, oder eine R'-CO-O-(R''CH)-O-CO-Gruppe, wobei

R' eine Alkylgruppe mit 1 bis 4 Kohlenstoffatomen oder eine Cycloalkylgruppe mit 5 oder 6 Kohlenstoffatomen und

R'' ein Wasserstoffatom oder eine Methylgruppe darstellen,

$X_1$ eine Alkylengruppe mit 1 oder 2 Kohlenstoffatomen oder eine -C(=NH)-Gruppe,

$X_2$ eine Phenylen-, Pyridinylen-, Pyrazinylen-, Pyrimidinylen- oder Pyridazinylengruppe, die jeweils im Kohlenstoffgerüst durch ein Fluor-, Chlor-, Brom- oder Jodatom, durch eine Alkyl-, Amino-, Hydroxy-, Alkoxy-, Pyrrolidino-, Piperidino-, Morpholino-, Thiomorpholino- oder N-Acetylpiperazinogruppe substituiert sein können und in denen der Alkyl- oder Alkoxyteil jeweils 1 oder 2 Kohlenstoffatome enthalten kann, oder eine Thiazolylengruppe,

$X_3$ die für $X_2$ vorstehend erwähnten Bedeutungen mit der Maßgabe besitzt, daß $X_3$ keine Thiazolylengruppe und mindestens einer der Reste $X_2$ oder $X_3$ einen der vorstehend erwähnten heteroaromatischen Reste darstellt, und $X_3$ eine 1,2,4-Triazinylengruppe, wobei in den bei der Definition des Restes $X_3$ vorstehend erwähnten heteroaromatischen Ringen gleichzeitig eine oder zwei -N=CH-Gruppen durch eine oder zwei $-NR_5$-CO-Gruppen ersetzt sein können, in der

$R_5$ ein Wasserstoffatom, eine Alkyl-, Phenyl-, Benzyl-, Aminocarbonylalkyl-, Alkylaminocarbonylalkyl-, Dialkylaminocarbonylalkyl-, $R_2CO$-alkyl- oder $R_3CO-CHR_4$-NHCO-alkyl-Gruppe darstellt, in denen der Alkylteil jeweils 1 bis 3 Kohlenstoffatome enthalten kann, $R_2$ eine Pyrrolidino-, Piperidino- oder Hexamethyleniminogruppe, in welcher die Methylengruppe in 4-Stellung einer Piperidinogruppe zusätzlich durch eine -O-, -S-, -NH-, -N(Methyl)-, -NCHO- oder $-N(COCH_3)$-Gruppe ersetzt sein kann, und $R_5$ zusätzlich in einem bei der Definition des Restes $X_3$ vorstehend erwähnten Ringe, die eine $-NR_5$-CO-Gruppe enthalten, eine Bindung zu dem Rest $X_2$ oder auch, wenn $Y_1$ eine Bindung darstellt, zu dem Rest $Y_2$ bedeuten kann,

$R_3$ eine Hydroxygruppe oder eine Alkoxygruppe mit 1 bis 5 Kohlenstoffatomen, in der der Alkoxyteil in 1- oder 2-Stellung durch eine Aryl- oder Pyridylgruppe oder in 2-Stellung durch eine Morpholino- oder Thiomorpholinogruppe substituiert sein kann, und

$R_4$ eine Alkylgruppe mit 1 bis 4 Kohlenstoffatomen, die in 1- oder 2-Stellung durch eine Phenyl- oder $R_3CO$-Gruppe substituiert sein kann, wobei $R_3$ wie eingangs definiert ist und der Phenylrest durch ein Chlor- oder Bromatom, durch eine Hydroxy- oder Aminogruppe substituiert sein kann, darstellen,

$Y_1$ eine Bindung, eine -O-, -CO-, -NH-, $-NCH_3$-, -CONH-, $-CONCH_3$- oder $-SO_2NH$-Gruppe oder auch eine $-OCH_2CO$-Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylengruppe mit 2 bis 6 Kohlenstoffatomen, wobei die Doppelbindung nicht mit einem Sauerstoff-, Schwefel- oder Phosphoratom der Reste $Y_1$, $Y_3$ oder E direkt verknüpft sein kann, eine Cyclohexylengruppe oder eine gegebenenfalls durch ein Fluor-, Chlor- oder Bromatom oder durch eine Methylgruppe substituierte Phenylengruppe,

$Y_3$ eine Bindung, eine -CO-, -CONH- oder $-CONCH_3$-Gruppe oder auch, wenn ein Heteroatom des Restes $Y_1$ nicht an dasselbe Kohlenstoffatom des Restes $Y_2$ gebunden ist wie der Rest $Y_3$, eine -O-, -NH-, $-N(COCH_3)$-, -N(Benzoyl)- oder $-N(SO_2CH_3)$-Gruppe,

eine Gruppe der Formeln

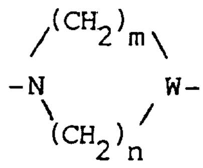

oder

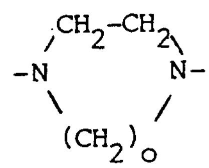

in denen

W eine >CH-, >C(OH)-, >CH-O-, >C=CH- oder >C($CH_2COR_3$)-Gruppe, wobei $R_3$ wie vorstehend erwähnt definiert ist,

m und n jeweils die Zahlen 1, 2 oder 3, wobei jedoch m + n die Zahl 3 oder 4 darstellen muß, und

o die Zahl 2 bedeuten, wobei in den vorstehend erwähnten Ringen zusätzlich eine Methylengruppe durch eine Carbonylgruppe ersetzt sein kann oder auch, falls die Methylengruppe nicht benachbart zu einem Stickstoffatom steht, durch eine Hydroxygruppe substituiert sein kann, sowie generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,

$Y_4$ eine Bindung, eine geradkettige oder verzweigte Alkylengruppe mit 1 bis 4 Kohlenstoffatomen oder eine gegebenenfalls durch ein Fluor-, Chlor- oder Bromatom oder durch eine Methylgruppe substituierte Phenylengruppe und

E eine Sulfo-, 5-Tetrazolyl-, Phosphono-, O-Methyl-phosphono-, R'-CO-O-(R''CH)-O-CO-, R'''CO- oder R'O-CO-O-(R''CH)-O-CO-Gruppe, wobei

R' und R'' wie vorstehend erwähnt definiert sind und

R''' eine Hydroxygruppe, eine Alkoxygruppe mit 1 bis 5 Kohlenstoffatomen, in der der Alkoxyteil in 1- oder 2-Stellung durch eine Phenyl- oder Pyridylgruppe oder in 2- oder 3-Stellung durch eine Pyrrolidino-, Piperidino-, Hexamethylenimino-, Morpholino- oder Thiomorpholinogruppe substituiert sein kann, eine Cycloalkoxygruppe mit 4 bis 7 Kohlenstoffatomen, eine Cycloalkylalkoxygruppe mit 4 bis 7 Kohlenstoffatomen im Cycloalkylteil und 1 bis 3 Kohlenstoffatomen im Alkoxyteil, oder eine Phenylallyloxygruppe darstellen,

und mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann, bedeuten,

deren Tautomere, deren Stereoisomere, deren Gemische und deren Salze.

Besonders bevorzugte Verbindungen der obigen allgemeinen Formeln sind jedoch diejenigen, in denen

$R_1$ ein Wasserstoffatom, eine Alkylgruppe mit 1 bis 4 Kohlenstoffatomen, eine Alkoxycarbonylgruppe mit insgesamt 2 bis 5 Kohlenstoffatomen, eine Benzyloxycarbonyl-, Dimethylphosphoryl-, Diethylphosphoryl- oder R'-CO-O-(R''CH)-O-CO-Gruppe, wobei

R' eine Methyl- oder Ethylgruppe und

R'' ein Wasserstoffatom oder eine Methylgruppe darstellen,

$X_1$ eine Methylengruppe oder eine -C(=NH)-Gruppe,

$X_2$ eine Phenylen-, Pyridinylen-, Pyrazinylen-, Pyrimidinylen- oder Pyridazinylengruppe, die jeweils im Kohlenstoffgerüst durch ein Fluor-, Chlor- oder Bromatom oder durch eine Methyl-, Hydroxy-, Methoxy-, Ethoxy-, Amino-, Dimethylamino-, Pyrrolidino-, Piperidino-, Morpholino-, Thiomorpholino- oder N-Acetylpiperazinogruppe substituiert sein können, oder eine Thiazolylengruppe,

$X_3$ die für $X_2$ vorstehend erwähnten Bedeutungen mit der Maßgabe besitzt, daß $X_3$ keine Thiazolylengruppe und mindestens einer der Reste $X_2$ oder $X_3$ einen der vorstehend erwähnten heteroaromatischen Reste darstellt, und $X_3$ eine 1,2,4-Triazinylengruppe, wobei in den bei der Definition des Restes $X_3$ vorstehend erwähnten heteroaromatischen Ringen gleichzeitig eine -N=CH-Gruppe durch eine -$NR_5$-CO-Gruppe ersetzt sein kann, in der

$R_5$ ein Wasserstoffatom, eine Methyl-, Ethyl-, Phenyl-, Benzyl-, Aminocarbonylmethyl-, Dimethylaminocarbonylmethyl-, $R_2$CO-methyl- oder $R_3$CO-$CHR_4$-NHCO-methyl-Gruppe darstellt, wobei $R_2$ eine Morpholinogruppe,

$R_3$ eine Hydroxygruppe oder eine Alkoxygruppe mit 1 bis 3 Kohlenstoffatomen und

$R_4$ eine Methyl- oder Benzylgruppe und $R_5$ zusätzlich in einem bei der Definition des Restes $X_3$ vorstehend erwähnten Ringe, die eine -$NR_5$-CO-Gruppe enthalten, eine Bindung zu dem Rest $X_2$ oder auch, wenn $Y_1$ eine Bindung darstellt, zu dem Rest $Y_2$ bedeuten kann, darstellen,

$Y_1$ eine Bindung, eine -O-, -CO-, -NH-, -$NCH_3$-, -CONH-, -$CONCH_3$- oder -$SO_2$NH-Gruppe oder auch eine -$OCH_2$CO-Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylengruppe mit 2 bis 6 Kohlenstoffatomen, wobei die Doppelbindung nicht mit einem Sauerstoff-, Schwefel- oder Phosphoratom der Reste $Y_1$, $Y_3$ oder E direkt verknüpft sein kann, eine Cyclohexylen- oder Phenylengruppe,
$Y_3$ eine Bindung, eine -CO-, -CONH- oder -$CONCH_3$-Gruppe oder auch, wenn ein Heteroatom des Restes $Y_1$ nicht an dasselbe Kohlenstoffatom des Restes $Y_2$ gebunden ist wie der Rest $Y_3$, eine -O-, -NH-, -N($COCH_3$)-, -N(Benzoyl)- oder -N($SO_2CH_3$)-Gruppe,
eine Gruppe der Formeln

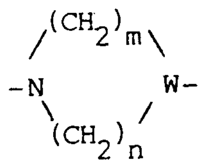

oder

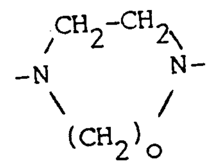

in denen
W eine >CH-, >C(OH)-, >CH-O-, >C=CH- oder >C($CH_2COR_3$)-Gruppe, wobei $R_3$ wie vorstehend erwähnt definiert ist,
m und n jeweils die Zahlen 1, 2 oder 3, wobei jedoch m + n die Zahl 3 oder 4 darstellen muß, und
o die Zahl 2 bedeuten, wobei in den vorstehend erwähnten Ringen zusätzlich eine Methylengruppe durch eine Carbonylgruppe ersetzt sein kann, und generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,
$Y_4$ eine Bindung, eine geradkettige oder verzweigte Alkylengruppe mit 1 bis 4 Kohlenstoffatomen oder eine Phenylengruppe und
E eine Sulfo-, 5-Tetrazolyl-, Phosphono-, O-Methyl-phosphono- oder R'''CO-Gruppe, wobei
R''' eine Hydroxygruppe, eine Alkoxygruppe mit 1 bis 4 Kohlenstoffatomen, eine Cycloalkoxy- oder Cycloalkoxymethoxygruppe mit jeweils 5 oder 6 Kohlenstoffatomen im Cycloalkoxyteil, eine Benzyloxy- oder Pyridylmethoxygruppe darstellt,
mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann, bedeuten,
deren Tautomere, deren Stereoisomere, deren Gemische und deren Salze.

Ganz besonders bevorzugte Verbindungen der obigen allgemeinen Formel I sind jedoch diejenigen, in denen
$R_1$ ein Wasserstoffatom oder eine Alkoxycarbonylgruppe mit insgesamt 2 oder 3 Kohlenstoffatomen,
$X_1$ eine Methylengruppe oder eine -C(=NH)-Gruppe,
$X_2$ eine Phenylengruppe,
$X_3$ eine Pyrimidinylen- oder Pyridazinylengruppe, die jeweils im Kohlenstoffgerüst durch eine Methoxy- oder Morpholinogruppe substituiert sein können, wobei in den vorstehend erwähnten heteroaromatischen Ringen gleichzeitig eine -N=CH-Gruppe durch eine -$NR_5$-CO-Gruppe ersetzt sein kann, wobei
$R_5$ ein Wasserstoffatom, eine Methyl-, Benzyl- oder Morpholinocarbonylmethylgruppe darstellt,
$Y_1$ eine Bindung, eine -O-, -CO-, -NH-, -$NCH_3$-, -CONH-oder -$CONCH_3$-Gruppe,
$Y_2$ eine Bindung, eine geradkettige oder verzweigte Alkylengruppe mit 1 bis 4 Kohlenstoff atomen, eine Cyclohexylen- oder Phenylengruppe,
$Y_3$ eine Bindung, eine -O-Gruppe oder eine Gruppe der Formel

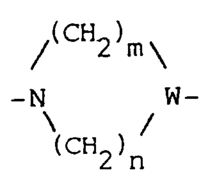

in der
W eine >CH- oder >CH-O-Gruppe,
m und n jeweils die Zahlen 1, 2 oder 3, wobei jedoch m + n die Zahl 4 darstellen muß, bedeuten, sowie generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,
$Y_4$ eine Bindung, eine Alkylengruppe mit 1 oder 2 Kohlenstoffatomen oder eine Phenylengruppe und
E eine Carboxygruppe oder eine Alkoxycarbonylgruppe mit insgesamt 2 bis 4 Kohlenstoffatomen, wobei mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann, bedeuten,
insbesondere jedoch diejenigen Verbindungen der allgemeinen Formel I, in denen
$R_1$ ein Wasserstoffatom oder eine Alkoxycarbonylgruppe mit insgesamt 2 oder 3 Kohlenstoffatomen,
$X_1$ eine Methylengruppe oder eine -C(=NH)-Gruppe,
$X_2$ eine Phenylengruppe,
$X_3$ eine gegebenenfalls durch eine Methoxygruppe substituierte Pyrimidinylengruppe, eine gegebenenfalls durch eine Methoxy- oder Morpholinogruppe substituierte Pyridazinylengruppe, eine Pyrimidinylen- oder Pyridazinylengruppe, in denen eine -N=CH-Gruppe durch eine -$NR_5$-CO-Gruppe ersetzt ist, wobei
$R_5$ ein Wasserstoffatom, eine Methyl-, Benzyl- oder Morpholinocarbonylmethylgruppe darstellt,
$Y_1$ eine Bindung, eine -CO-, -CONH- oder -$CONCH_3$-Gruppe,
$Y_2$ eine Bindung, eine geradkettige Alkylengruppe mit 3 oder 4 Kohlenstoffatomen oder eine Cyclohexylengruppe,
$Y_3$ eine Bindung oder eine Gruppe der Formel

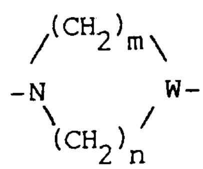

in der
W eine >CH-Gruppe,
m und n jeweils die Zahlen 1, 2 oder 3, wobei jedoch m + n die Zahl 4 darstellen muß, bedeuten,
$Y_4$ eine Bindung oder eine Methylengruppe und
E eine Carboxygruppe oder eine Alkoxycarbonylgruppe mit insgesamt 2 oder 3 Kohlenstoffatomen, wobei mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann, bedeuten.

Besonders bevorzugt sind hierbei folgende Verbindungen:
6-(4-Amidinophenyl)-4-[(trans-4-carboxycyclohexyl)-aminocarbonyl]-2-methyl-(2H)-pyridazin-3-on,
6-(4-Amidinophenyl)-4-[[trans-4-(methoxycarbonyl)-cyclohexyl]-aminocarbonyl]-2-methyl-(2H)-pyridazin-3-on,
6-[4-[N-(Methoxycarbonyl)-amidino]-phenyl]-4-[[trans-4-(methoxycarbonyl)-cyclohexyl]-aminocarbonyl]-2-methyl-(2H)-pyridazin-3-on,
6-[4-[N-(Ethoxycarbonyl)-amidino]-phenyl]-4-[[trans-4-(methoxycarbonyl)-cyclohexyl]-aminocarbonyl]-2-methyl-(2H)-pyridazin-3-on,
2-(4-Amidinophenyl)-5-[4-(carboxymethyl)-piperidinocarbonyl]-4-methoxy-pyrimidin,
2-(4-Amidinophenyl)-4-methoxy-5-[4-[(methoxycarbonyl)-methyl]-piperidinocarbonyl]-pyrimidin,
4-Methoxy-2-[4-[N-(methoxycarbonyl)-amidino]-phenyl]-5-[4-[(methoxycarbonyl)-methyl]-piperidinocarbonyl]-pyrimidin,
6-(4-Amidinophenyl)-4-[N-(trans-4-carboxycyclohexyl)-N-methyl-aminocarbonyl]-2-methyl-(2H)-pyridazin-3-on,
6-(4-Amidinophenyl)-4-[N-[trans-4-(methoxycarbonyl)-cyclohexyl]-N-methyl-aminocarbonyl]-2-methyl-(2H)-pyridazin-3-on,
3-(4-Amidinophenyl)-5-[(trans-4-carboxycyclohexyl)-aminocarbonyl]-pyridazin,
3-(4-Amidinophenyl)-5-[[trans-4-(methoxycarbonyl)-cyclohexyl]-aminocarbonyl]-pyridazin,
6-(4-Amidinophenyl)-4-[(trans-4-carboxycyclohexyl)-aminocarbonyl]-2-[(morpholinocarbonyl)-methyl]-(2H)-pyridazin-3-on und
6-(4-Amidinophenyl)-4-[[trans-4-(methoxycarbonyl)-cyclohexyl]-aminocarbonyl]-2-[(morpholinocarbonyl)-methyl]-(2H)-pyridazin3-on,
deren Tautomere und deren Salze.

Erfindungsgemäß erhält man die neuen Verbindungen der allgemeinen Formel I beispielsweise nach folgenden an und für sich bekannten Verfahren:

a) Zur Herstellung von Verbindungen der allgemeinen Formel I, in der E eine Carboxygruppe darstellt: Überführung einer Verbindung der allgemeinen Formel

$R_1NH - X_1 - X_2 - X_3 - Y_1 - Y_2 - Y_3 - Y_4 - E'$ ,(II)

in der
$R_1$, $X_1$ bis $X_3$ und $Y_1$ bis $Y_4$ wie eingangs definiert sind und E', das an ein Kohlenstoffatom gebunden ist, eine mittels Hydrolyse, Behandlung mit Säuren, Thermolyse oder Hydrogenolyse in eine Carboxygruppe überführbare Gruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe, Ester mit Aralkanolen, z.B. der Benzylester, mittels Hydrogenolyse in eine Carboxylgruppe, und Bis-(alkoxycarbonyl)methylgruppen mittels Hydrolyse oder Behandlung mit einer Säure in eine Bis-(hydroxycarbonyl)methylgruppe, welche anschließend decarboxyliert wird, übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Methanol, Wasser/Methanol, Äthanol, Wasser/Äthanol, Wasser/Isopropanol, Wasser/Tetrahydrofuran oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden. Bedeutet E' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Bedeutet E' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10°C und 120°C, z.B. bei Temperaturen zwischen 0 und 60°C, oder auch thermisch gegebenenfalls in einem inerten Losungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet E' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe oder eine Benzyloxygruppe zur Hydroxygruppe mitreduziert oder eine Benzyloxycarbonylamidinogruppe in die Amidinogruppe übergeführt werden.

b) Zur Herstellung von Verbindungen der allgemeinen Formel I, in der die $R_1NH-X_1$-Gruppe eine Amidinogruppe darstellt, in der $R_1$ ein Wasserstoffatom, eine Hydroxy-, Alkyl- oder Aminogruppe bedeutet:
Umsetzung einer gegebenenfalls im Reaktionsgemisch gebildeten Verbindung der allgemeinen Formel

$Z_1 - C(=NH) - X_2 - X_3 - Y_1 - Y_2 - Y_3 - Y_4 - E$ ,(III)

in der

$X_2$, $X_3$, $Y_1$ bis $Y_4$ und E wie eingangs definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_a$ - $NH_2$ ,(IV)

in der
$R_a$ ein Wasserstoffatom, eine Hydroxy-, Alkyl- oder Aminogruppe bedeutet, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise Ammoniumcarbonat oder Ammoniumacetat durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50°C, vorzugsweise jedoch bei Temperaturen zwischen 0 und 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

c) Zur Herstellung von Verbindungen der allgemeinen Formel I, in der die $R_1$NH-$X_1$-Gruppe eine Aminoalkylgruppe darstellt:
Reduktion einer Verbindung der allgemeinen Formel

NC - $X_1'$ - $X_2$ - $X_3$ - $Y_1$ - $Y_2$ - $Y_3$ - $Y_4$ - E ,(V)

in der
$X_2$, $X_3$, $Y_1$ bis $Y_4$ und E wie eingangs definiert sind und $X_1'$ eine Bindung oder eine Alkylengruppe mit 1 oder 2 Kohlenstoffatomen darstellt.

Die Reduktion wird vorzugsweise in einem geeigneten Lösungsmittel wie Methanol, Methanol/Wasser, Methanol/Ammoniak, Methanol/Wasser/Ammoniak, Methanol/Salzsäure, Ethanol, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid oder Lithiumborhydrid bei Temperaturen zwischen 0 und 100°C, vorzugsweise bei Temperaturen zwischen 20 und 80°C, durchgeführt.

d) Zur Herstellung von Verbindungen der allgemeinen Formel I, in der $R_1$ eine Alkoxycarbonyl-, Aralkoxycarbonyl-, Aryloxycarbonyl-, Alkylcarbonyl- oder Arylcarbonylgruppe, in denen der Alkyl- und Alkoxyteil jeweils 1 bis 4 Kohlenstoffatome enthalten kann, eine Alkenyloxycarbonylgruppe mit insgesamt 4 bis 6 Kohlenstoffatomen, eine Phosphono-, O-Alkylphosphono-, Dialkylphosphoryl- oder R'-CO-O-(R"CH)-O-CO-Gruppe darstellt, wobei R' und R" wie eingangs definiert sind:
Umsetzung einer Verbindung der allgemeinen Formel

$H_2N$ - $X_1$ - $X_2$ - $X_3$ - $Y_1$ - $Y_2$ - $Y_3$ - $Y_4$ - E ,(VI)

in der
$X_1$ bis $X_3$, $Y_1$ bis $Y_4$ und E wie eingangs definiert sind, mit einer Verbindung der allgemeinen Formel

$Z_2$ - $R_b$ ,(VII)

in der
$R_b$ eine Alkoxycarbonyl-, Aralkoxycarbonyl-, Aryloxycarbonyl-, Alkylcarbonyl- oder Arylcarbonylgruppe, in denen der Alkyl- und Alkoxyteil jeweils 1 bis 4 Kohlenstoffatome enthalten kann, eine Alkenyloxycarbonylgruppe mit insgesamt 4 bis 6 Kohlenstoffatomen, eine R'-CO-O-(R"CH)-O-CO- oder Dialkylphosphorylgruppe darstellt, wobei R' und R'' wie eingangs definiert sind, und

$Z_2$ eine nukleophile Austrittsgruppe wie ein Halogenatom, z. B. ein Chlor- oder Bromatom, oder eine gegebenenfalls substituierte Phenoxygruppe, z. B. eine p-Nitro-phenoxygruppe, darstellen, und gegebenenfalls anschließende Abspaltung einer oder zweier Alkylreste von einer so erhaltenen Dialkylphosphorylverbindung.

Die Umsetzung wird zweckmäßigerweise in einem Lösungsmittel oder Lösungsmittelgemisch wie Wasser, Tetrahydrofuran, Tetrahydrofuran/Wasser, Dioxan, Dioxan/Wasser, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperatu- ren zwischen -30 und 100°C, vorzugsweise jedoch bei Temperaturen zwischen -10 und 80°C, durchgeführt.

Die Abspaltung eines Alkylrestes von einer so erhaltenen Dialkylphosphorylverbindung erfolgt beispielsweise mit Natriumjodid in einem Lösungsmittel wie Aceton, Ethylmethylketon, Acetonitril oder Dimethylformamid bei Temperaturen zwischen 40 und 150°C, vorzugsweise jedoch bei Temperaturen zwischen 60 und 100°C.

Die Abspaltung beider Alkylreste von einer so erhaltenen Dialkylphosphorylverbindung erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen O°C und der Siedetemperatur des Reaktionsgemisches, vorzugsweise jedoch bei Temperaturen zwischen 20 und 60°C.

e) Zur Herstellung von Verbindungen der allgemeinen Formel I, in der E eine R'''CO-Gruppe darstellt, wobei R''' wie eingangs definiert ist:

Umsetzung einer Verbindung der allgemeinen Formel

$$R_1NH - X_1 - X_2 - X_3 - Y_1 - Y_2 - Y_3 - Y_4 - E'' \quad ,(VIII)$$

in der

$R_1$, $X_1$ bis $X_3$ und $Y_1$ bis $Y_4$ wie eingangs definiert sind und

E'' eine Carboxy- oder Alkoxycarbonylgruppe darstellt, mit einem Alkohol der allgemeinen Formel

$$HO - R''' \quad ,(IX)$$

in der

R''' wie eingangs definiert ist.

Die Umsetzung wird zweckmäßigerweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, Dimethylaminopyridin oder 1-Hydroxy-benzotriazol, N,N'-Carbonyldiimidazol- oder N,N'-Thionyldiimidazol oder Triphenylphosphin/Tetrachlorkohlenstoff, zweckmäßigerweise bei Temperaturen zwischen 0 und 150°C, vorzugsweise bei Temperaturen zwischen 0 und 50°C, durchgeführt.

Die Umsetzung einer entsprechenden Alkoxyverbindung der allgemeinen Formel VIII mit einem Alkohol der allgemeinen Formel IX wird vorzugsweise in dem betreffenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 100°C, vorzugsweise bei Temperaturen zwischen 20 und 80°C, durchgeführt.

f) Zur Herstellung von Verbindungen der allgemeinen Formel I, in der $Y_1$ eine -CO- oder -$CONR_6$-Gruppe darstellt:

Umsetzung einer Verbindung der allgemeinen Formel

$$R_1NH - X_1 - X_2 - X_3 -COOH \quad ,(X)$$

in der

$R_1$ und $X_1$ bis $X_3$ wie eingangs definiert sind, mit einer Verbindung der allgemeinen Formel

$U - Y_2 - Y_3 - Y_4 - E$ ,(XI)

in der

$Y_2$ bis $Y_4$ und E wie eingangs definiert sind und

U, falls $Y_2$ keine Bindung darstellt, eine $-NR_7$-Gruppe, wobei $R_7$ wie eingangs definiert ist, oder, wenn $Y_3$ eine der eingangs erwähnten cyclischen Iminogruppen und $Y_2$ eine Bindung darstellen, auch ein Wasserstoffatom bedeuten, oder mit deren reaktionsfähigen Derivaten.

Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid,N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die Acylierung wird jedoch wie vorstehend beschrieben vorzugsweise mit einem entsprechenden Säurehalogenid oder Säureanhydrid durchgeführt, wobei diese auch ohne Lösungsmittel durchgeführt werden kann.

g) Zur Herstellung der Verbindungen der allgemeinen Formel I, in der E eine R'-CO-O-(R''CH)-O-CO-, R'O-CO-O-(R''CH)-O-CO- oder R'''CO-Gruppe darstellt, wobei R' bis R''' wie eingangs definiert sind:

Umsetzung einer Verbindung der allgemeinen Formel

$R_1NH - X_1 - X_2 - X_3 - Y_1 - Y_2 - Y_3 - Y_4 - COOH$ ,(XII)

in der

$R_1$, $X_2$ bis $X_3$ und $Y_1$ bis $Y_4$ wie eingangs definiert sind, mit einer Verbindung der allgemeinen Formel

$Z_3 - R_c$ ,(XIII)

in der

$R_c$ eine R'-CO-O-(R''CH)-, R'O-CO-O-(R''CH)- oder R'''-Gruppe, wobei R' bis R''' wie eingangs definiert sind, und

$Z_3$ eine nukleophile Austrittsgruppe wie ein Halogenatom, z. B. ein Chlor- oder Bromatom, darstellen.

Die Umsetzung wird zweckmäßigerweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe und

als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und bei einem wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50 ° C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 ° C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und chirale Verbindungen in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese in den Beispielen I bis XXIII beschrieben werden.

Wie bereits eingangs erwähnt, weisen die neuen Heterobiaryle der allgemeinen Formel I und deren Salze, insbesondere deren physiologisch verträgliche Salze mit anorganischen oder organischen Säuren oder Basen, wertvolle Eigenschaften auf. So zeigen die neuen Verbindungen der allgemeinen Formel I, in denen $R_1$ eine gegebenenfalls in vivo abspaltbare Gruppe, z.B. eine Alkoxycarbonylgruppe, und E eine Carboxyl-, Phosphono-, O-Alkyl-phosphono- oder 5-Tetrazolylgruppe oder eine in vivo in eine Carboxyl-, Sulfo-, Phosphono-, O-Alkyl-phosphono- oder Tetrazolylgruppe überführbare Gruppe, z.B. eine durch eine Alkoxygruppe substituierte Carbonylgruppe, darstellen neben einer entzündungshemmenden und den

Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. Fibrinogen-Bindung an Humanthrombozyten

Das durch Funktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg $^{125}$J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37° C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[µM] | Hemmung der Plättchenaggregation $EC_{50}$[µM] |
|---|---|---|
| 1 | 0,13 | 0,42 |
| 1(3) | 0,20 | 0,39 |
| 1(4) | 0,037 | 0,54 |
| 1(7) | 4,40 | 12,00 |
| 1(8) | 1,70 | 39,00 |
| 1(11) | 0,58 | 1,20 |
| 1(14) | 0,033 | 0,10 |
| 1(16) | 0,055 | 0,16 |
| 1(18) | 0,033* | 0,31 |
| 1(20) | 0,057 | 0,29 |
| 1(21) | 0,062 | 0,32 |
| 1(30) | 0,033* | 0,10 |
| 2 | 1,90 | 0,36 |
| 2(1) | 3,20 | 1,90 |
| 2(15) | 52,00 | 5,20 |
| 2(17) | 2,30 | 1,30 |
| 2(21) | 7,80 | 0,45 |
| 2(31) | 0,40 | 0,61 |
| 3 | 2,20 | >300,00 |
| 3(1) | 3,00 | >300,00 |
| 4 | 0,37 | 0,79 |
| 5 | 6,20 | 3,00 |

* $^{125}$I Fibrinogen wurde durch [$^{3}$H]-(3S,5S)-5-[(4'-Amidino-4-biphenylyl)-oxymethyl]-3-carboxymethyl-2-pyrrolidon ersetzt.

Außerdem zeigt beispielsweise an der Ratte die Verbindung des Beispiels 3 zwei Stunden nach oraler Gabe von 10 mg/kg in einem ex vivo-Test, welcher mit Rattenplasma in Gegenwart von Humanthrombozyten durchgeführt wird, eine antiaggregatorische Wirkung.

Die erfindungsgemäßen Verbindungen sind gut verträglich, da beispielsweise bei intravenöser Gabe von 30 mg/kg an jeweils drei Mäusen bei den Verbindungen der Beispiele 1, 1(4), 1(16), 1(20), 3 und 4 keine Tiere gestorben waren.

Auf Grund ihrer Hemmwirkung auf Zell-Zell- bzw. Zell-Matrix-Wechselwirkungen eignen sich die neuen Heterobiaryle der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose, der Metastasierung von Tumoren und der Therapie genetisch bedingter oder auch erworbener Störungen der Interaktion von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 20 mg/kg Körpergewicht, vorzugsweise bei 1 µg bis 10 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen

Beispiel I

3-(4-Cyanobenzoyl)-2-hydroxy-propionsäure

25 g 4-Cyano-acetophenon und 24 g Glyoxylsäure-monohydrat werden im Wasserstrahl-Vakuum 5 Stunden bei 95 ° C gerührt. Man erhält ein zähes Öl, welches in einem Gemisch aus gesättigter Natriumhydrogencarbonat-Lösung und Essigester gelöst wird. Die organische Phase wird abgetrennt, die wässrige Phase mit 2N Salzsäure angesäuert und mehrmals mit Essigester extrahiert. Die vereinigten Essigester-Phasen werden mit Wasser gewaschen, getrocknet und eingedampft. Der Rückstand wird mit Ether verrieben, abgesaugt und als Rohprodukt weiter umgesetzt.
Ausbeute: 18,2 g (48 % der Theorie),
$R_f$-Wert: 0,25 (Kieselgel; Methylenchlorid/Methanol/Essigsaure = 8:2:0,2)

Beispiel II

6-(4-Cyanophenyl)-(2H)-pyridazin-3-on

19,2 g 3-(4-Cyanobenzoyl)-2-hydroxy-propionsäure und 20 ml 80%ige Hydrazinlösung in 200 ml Essigsäure werden 2 Stunden unter Rückfluß erhitzt. Man läßt abkühlen, saugt den Niederschlag ab und wäscht mit Essigsäure und Ether.
Ausbeute: 10,3 g (60 % der Theorie),
$R_f$-Wert: 0,32 (Kieselgel; Methylenchlorid/Methanol = 19:1)

Beispiel III

3-Chlor-6-(4-cyanophenyl)-pyridazin

Eine Suspension von 27,6 g 6-(4-Cyanophenyl)-(2H)-pyridazin-3-on in 200 ml Phosphoroxychlorid wird 2 Stunden unter Rückfluß erhitzt. Die Reaktionslösung wird in 1 l Wasser eingerührt, der Niederschlag abgesaugt, mit Wasser nachgewaschen und getrocknet.
Ausbeute: 25,7 g (85 % der Theorie),
Schmelzpunkt: 237-240 ° C,
$R_f$-Wert: 0,75 (Kieselgel; Methylenchlorid/Methanol = 19:1)
Analog wird folgende Verbindung erhalten:

(1) 3-Chlor-6-(4-cyanophenyl)-4-(ethoxycarbonyl)-pyridazin Nach Einrühren der Reaktionslösung in Wasser wird die wässrige Phase mit Essigester extrahiert. Die organische Phase wird getrocknet, eingedampft und der Rückstand chromatographiert.
$R_f$-Wert: 0,37 (Kieselgel; Cyclohexan/Essigester = 2:1)

Beispiel IV

2-(4-Bromphenyl)-2-oxo-ethylmalonsäure-diethylester

Zu einer Lösung von 165 ml Malonsäure-diethylester in 600 ml Dimethylformamid gibt man 124 g Kalium-tert.butylat. Unter Kühlung im Wasserbad werden 307 g 4-Bromphenacylbromid portionsweise zugegeben, wobei sich die Reaktionslösung auf ca. 70 ° C erwärmt. Man rührt 2 Stunden bei Raumtemperatur, gießt die Reaktionslösung in verdünnte Kochsalzlösung und extrahiert mit Essigester. Die organische Phase wird mit Wasser gewaschen, über Natriumsulfat getrocknet und über Aktivkohle filtriert. Nach Entfernen des Lösungsmittels am Rotationsverdampfer erhält man 398 g eines roten Öls, welches ohne Reinigung weiter umgesetzt wird.
$R_f$-Wert: 0,40 (Kieselgel; Cyclohexan/Essigester = 5:1)

Beispiel V

6-(4-Bromphenyl)-4, 5-dihydro-4-ethoxycarbonyl-(2H)-pyridazin-3-on

Zu einer Lösung von 398 g 2-(4-Bromphenyl)-2-oxo-ethylmalonsäure-diethylester in 1,5 l Eisessig gibt man 240 ml 80%ige Hydrazin-Lösung und erhitzt 2 Stunden zum Rückfluß. Beim Abkühlen der Reaktionslösung entsteht ein Niederschlag, der abgenutscht, mit Wasser gewaschen und getrocknet wird. Um weiteres Produkt zu isolieren wird die Mutterlauge mit Wasser versetzt, der Niederschlag abgenutscht und aus Eisessig umkristallisiert.
Ausbeute: 213 g (61 % oder Theorie),
$R_f$-Wert: 0,31 (Kieselgel; Cyclohexan/Essigester = 2:1)

Beispiel VI

6-(4-Bromphenyl)-4-ethoxycarbonyl-(2H)-pyridazin-3-on

Zu einer Suspension von 213 g 6-(4-Bromphenyl)-4,5-dihydro-4-ethoxycarbonyl-(2H)-pyridazin-3-on in 2,0 l Eisessig tropft man eine Lösung von 40 ml Brom in 100 ml Eisessig und rührt 45 Minuten bei Raumtemperatur. Man verdünnt mit 2,0 l Wasser und zerstört überschüssiges Brom mit verdünnter Natriumsul- fit-Lösung. Der Niederschlag wird abgenutscht, mit Wasser gewaschen und getrocknet.
Ausbeute: 206 g (97 % der Theorie),
$R_f$-Wert: 0,39 (Kieselgel; Methylenchlorid/Methanol = 15:1)

Beispiel VII

6-(4-Cyanophenyl)-4-ethoxycarbonyl-(2H)-pyridazin-3-on

Zu einer Lösung von 24,6 g 6-(4-Bromphenyl)-4-ethoxycarbonyl-(2H)-pyridazin-3-on in 100 ml Dimethylformamid gibt man 6,9 g Kupfer(I)cyanid und erhitzt 6 Stunden unter Rückfluß. Die Reaktionslösung wird abgekühlt und in Wasser eingerührt. Der Niederschlag wird abgenutscht und getrocknet. Man erhält 23 g Rohprodukt, welches ohne Reinigung weiter umgesetzt wird.
$R_f$-Wert: 0,39 (Kieselgel; Methylenchlorid/Methanol = 15:1)

Beispiel VIII

6-(4-Cyanophenyl)-4-ethoxycarbonyl-2-methyl-(2H)-pyridazin-3-on

Eine Suspension aus 18 g rohem 6-(4-Cyanophenyl)-4-ethoxycarbonyl-(2H)-pyridazin-3-on, 7,5 ml Methyliodid, 2,0 g Methyltrioctylammoniumchlorid in 200 ml 2M Kaliumhydrogencarbonat-Lösung, 500 ml Tetrahydrofuran und 1,6 l Toluol wird 16 Stunden bei Raumtemperatur gerührt. Es werden 3 ml Methyliodid hinzugegeben und weitere 4 Stunden gerührt. Der Niederschlag wird abgenutscht und das Filtrat mit Wasser gewaschen. Die organische Phase wird über Natriumsulfat getrocknet, das Lösungsmittel im Vakuum abgedampft und das Rohprodukt über Kieselgel chromatographiert.
Ausbeute: 11,2 g (59 % oder Theorie),
$R_f$-Wert: 0,49 (Kieselgel; Methylenchlorid/Methanol = 9:1)
Analog werden folgende Verbindungen erhalten:

(1) 2-Benzyl-6-(4-cyanophenyl)-4-ethoxycarbonyl-(2H)-pyridazin-3-on
Es wird Benzylbromid eingesetzt.
Schmelzpunkt: 151-153 °C
$R_f$-Wert: 0,55 (Kieselgel; Methylenchlorid/Methanol = 9:1)

(2) 6-(4-Cyanophenyl)-4-ethoxycarbonyl-2-[(morpholinocarbonyl)-methyl]-(2H)-pyridazin-3-on
Es wird 1-Chlor-essigsäuremorpholid eingesetzt.
Schmelzpunkt: 186-192 °C
$R_f$-Wert: 0,57 (Kieselgel; Methylenchlorid/Methanol = 9:1)

Beispiel IX

4-Carboxy-6-(4-cyanophenyl)-2-methyl-(2H)-pyridazin-3-on

Zu einer Lösung von 12,0 g 6-(4-Cyanophenyl)-4-ethoxycarbonyl-2-methyl-(2H)-pyridazin-3-on in 200 ml Tetrahydrofuran gibt man eine Lösung von 7,14 g Lithiumhydroxid-Monohydrat in 170 ml Wasser und kontrolliert die Umsetzung mittels Dünnschicht-Chromatographie. Nach 1,5 Stunden wird mit 1N Salzsäure angesäuert, das Tetrahydrofuran im Vakuum abgedampft, der Niederschlag abgenutscht und getrocknet.
Ausbeute: 11,2 g (quant.),
Schmelzpunkt: 190-194 ° C,
$R_f$-Wert: 0,33 (Kieselgel; Methylenchlorid/Methanol/konz. Ammoniaklösung = 4:1:0,25)
Analog werden folgende Verbindungen erhalten:

(1) 4-Carboxy-6-(4-cyanophenyl)-(2H)-pyridazin-3-on Schmelzpunkt: über 290 ° C

(2) 2-(4-Carbamoyl-phenyl)-5-carboxy-(3H)-pyrimidin-4-on $R_f$-Wert: 0,06 (Kieselgel; Methylenchlorid/Methanol/konz. Ammoniaklösung = 2:1:0,25)

| | | | | | | |
|---|---|---|---|---|---|---|
| Ber.:<br>Gef.: | C | 55,60<br>55,45 | H | 3,50<br>3,53 | N | 16,21<br>16,36 |

(3) 4-[(trans-4-Carboxycyclohexyl)-aminocarbonyl]-6-(4-cyanophenyl)-2-methyl-(2H)-pyridazin-3-on
Schmelzpunkt: 283-286 ° C,
$R_f$-Wert: 0,39 (Kieselgel; Methylenchlorid/Methanol = 9:1)

(4) 4-Carboxy-3-chlor-6-(4-cyanophenyl)-pyridazin
Schmelzpunkt: 208-210 ° C (Zers.)
$R_f$-Wert: 0,74 (Kieselgel; Methylenchlorid/Methanol/konz. Ammoniaklösung = 4:1:0,25)

(5) 2-Benzyl-4-carboxy-6-(4-cyanophenyl)-(2H)-pyridazin-3-on
Schmelzpunkt: 254-256 ° C
$R_f$-Wert: 0,29 (Kieselgel; Methylenchlorid/Methanol/konz. Ammoniaklösung = 4:1:0,25)

(6) 4-Carboxy-6-(4-cyanophenyl)-2-[(morpholinocarbonyl)-methyl]-(2H)-pyridazin-3-on
Schmelzpunkt: Sinterung ab 215 ° C
$R_f$-Wert: 0,16 (Kieselgel; Methylenchlorid/Methanol/konz. Ammoniaklösung = 4:1:0,25)

(7) 2-Benzyl-4-[(trans-4-carboxycyclohexyl)-aminocarbonyl]-6-(4-cyanophenyl)-(2H)-pyridazin-3-on
Schmelzpunkt: 215-218 ° C
$R_f$-Wert: 0,51 (Kieselgel; Methylenchlorid/Methanol = 9:1)

Beispiel X

6-(4-Cyanophenyl)-4-[[trans-4-(methoxycarbonyl)-cyclohexyl]-aminocarbonyl]-2-methyl-(2H)-pyridazin-3-on

Eine Lösung von 2,0 g 4-Carboxy-6-(4-cyanophenyl)-2-methyl-(2H)-pyridazin-3-on und 1,2 g N-Methylmorpholin in 160 ml absolutem Tetrahydrofuran wird auf -20 ° C gekühlt und mit 1,1 g Chlorameisensäure-isobutylester versetzt. Es wird eine Stunde bei -20 ° C gerührt, anschließend auf -40 ° C abgekühlt und eine Lösung von 1,57 g trans-4-Amino-cyclohexancarbonsäure-methylester in 20 ml absolutem Tetrahydrofuran zugegeben. Man entfernt das Kühlbad und rührt 3 Stunden bei Raumtemperatur. Die Reaktionslösung wird in 400 ml 0,1N Salzsäure gegossen und die wässrige Phase mehrmals mit Essigester extrahiert. Trocknen der organischen Phase über Natriumsulfat und Abdampfen des Lösungsmittels liefern einen gelblichen Feststoff, welcher mit Methanol verrieben wird. Man nutscht ab, trocknet und erhält 2,5 g (81 % der Theorie) Produkt.
Schmelzpunkt: 210-215 ° C,
$R_f$-Wert: 0,85 (Kieselgel; Methylenchlorid/Methanol = 9:1)
Analog werden folgende Verbindungen erhalten:

(1) 6-(4-Cyanophenyl)-4-[[2-(ethoxycarbonyl)-ethyl]-aminocarbonyl]-2-methyl-(2H)-pyridazin-3-on
Es wird $\beta$-Alanin-ethylester-hydrochlorid mit einem Equivalent N-Methylmorpholin eingesetzt.
Schmelzpunkt: 132-136 ° C,
$R_f$-Wert: 0,39 (Kieselgel; Methylenchlorid/Methanol = 20:1)

(2) 6-(4-Cyanophenyl)-4-[[3-(methoxycarbonyl)-propyl]-aminocarbonyl]-2-methyl-(2H)-pyridazin-3-on
Es wird 4-Aminobuttersäure-ethylester-hydrochlorid mit einem Equivalent N-Methylmorpholin eingesetzt.

Schmelzpunkt: 130-132°C,
$R_f$-Wert: 0,42 (Kieselgel; Methylenchlorid/Methanol = 15:1)
(3) 6-(4-Cyanophenyl)-4-[[4-[(methoxycarbonyl)-methyl]-phenyl]-aminocarbonyl]-2-methyl-(2H)-pyridazin-3-on
Es wird frisch destillierter 4-Aminophenyl-essigsäure-methylester eingesetzt.
Schmelzpunkt: 235-238°C,
(4) 6-(4-Cyanophenyl)-4-[[cis-4-(methoxycarbonyl)-cyclohexyl]-aminocarbonyl]-2-methyl-(2H)-pyridazin-3-on
Schmelzpunkt: 190-192°C,
$R_f$-Wert: 0,77 (Kieselgel; Methylenchlorid/Methanol = 15:1)
(5) 6-(4-Cyanophenyl)-4-[[N-[trans-4-(methoxycarbonyl)-cyclohexyl]-N-methyl]-aminocarbonyl)-2-methyl-(2H)-pyridazin-3-on Es wird trans-4-(Methylamino)-cyclohexan-carbonsäuremethylester eingesetzt.
Schmelzpunkt: 228-230°C,
$R_f$-Wert: 0,48 (Kieselgel; Methylenchlorid/Methanol = 9:1)
(6) 3-Chlor-6-(4-cyanophenyl)-4-[[trans-4-(methoxycarbonyl)-cyclohexyl]-aminocarbonyl]-pyridazin
Schmelzpunkt (nach Chromatographie): 232-234°C,
$R_f$-Wert: 0,24 (Kieselgel; Methylenchlorid/Methanol = 20:1)
(7) 2-Benzyl-6-(4-cyanophenyl)-4-[[trans-4-(methoxycarbonyl)-cyclohexyl]-aminocarbonyl]-(2H)-pyridazin-3-on
Schmelzpunkt: 217-220°C,
$R_f$-Wert: 0,64 (Kieselgel; Methylenchlorid/Methanol = 9:1)

## Beispiel XI

### 6-(4-Cyanophenyl)-4-[(4-carboxybutyl)-aminocarbonyl]-2-methyl-(2H)-pyridazin-3-on

Eine Lösung von 530 mg 4-Carboxy-6-(4-cyanophenyl)-2-methyl-(2H)-pyridazin-3-on und 242 mg N-Methylmorpholin in 20 ml absolutem Tetrahydrofuran wird auf -15°C gekühlt und mit 273 mg Chlorameisensäure-isobutylester versetzt. Es wird eine Stunde bei -15°C gerührt. Anschließend wird eine 16 Stunden bei Raumtemperatur gerührte Lösung aus 234 mg 5-Amino-valeriansäure, 455 mg N-Methylmorpholin und 435 mg Trimethylsilylchlorid in 50 ml Tetrahydrofuran zugegeben. Nach 2 Stunden wird das Kühlbad entfernt und 3 Stunden bei Raumtemperatur gerührt. Die Reaktionslösung wird in gesättigte Natriumchlorid-Lösung gegossen, mit 2N Salzsäure angesäuert und die wässrige Phase mehrmals mit Essigester extrahiert. Trocknen der organischen Phase über Natriumsulfat und Abdampfen des Lösungsmittels liefern einen gelblichen Feststoff, welcher mit wenig Methylenchlorid verrieben wird. Man nutscht ab, trocknet und erhält 600 mg (85 % der Theorie) Produkt.
Schmelzpunkt: 194-197°C,
$R_f$-Wert: 0,51 (Kieselgel; Methylenchlorid/Methanol = 9:1)
Analog wird folgende Verbindung erhalten:
(1) 6-(4-Cyanophenyl)-4-[(4-carboxybutyl)-aminocarbonyl]-(2H)-pyridazin-3-on
$R_f$-Wert: 0,30 (Kieselgel; Methylenchlorid/Methanol = 9:1)

## Beispiel XII

### 4-Amidino-benzoesäureamid-hydrochlorid

Durch eine im Eis/Wasser-Bad gekühlte Lösung von 17,5 g 4-Cyano-benzoesäureamid in 700 ml Methanol leitet man 2 Stunden trockenes HCl-Gas (vorgeschaltete Waschflasche mit konzentrierter Schwefelsäure). Die Lösung wird bei Raumtemperatur gerührt und der Umsatz mittels Dünnschicht-Chromatographie kontrolliert. Nach vollständiger Umsetzung wird die Reaktionslösung bei einer Badtemperatur von 25-35°C im Rotationsverdampfer eingedampft, der Rückstand in 300 ml Methanol gelöst und die Lösung unter gutem Rühren mit 10,0 g Ammoniumcarbonat versetzt. Man rührt 16 Stunden bei Raumtemperatur, saugt den Niederschlag ab, wäscht mit wenig Wasser nach und trocknet im Vakuum. Ausbeute: 8,5 g (35 % der Theorie). Durch Einengen des Filtrats und Verreiben des Rückstands mit wenig Wasser, Abnutschen und Trocknen lassen sich weitere 8,8 g (37 % der Theorie) Produkt erhalten.
Schmelzpunkt: über 280°C,
$R_f$-Wert: 0,09 (Kieselgel; Methylenchlorid/Methanol/konz. Ammoniaklösung = 2:1:0,25)

Beispiel XIII

2-(4-Carbamoyl-phenyl)-5-ethoxycarbonyl-(3H)-pyrimidin-4-on

Unter Inertgasatmosphäre stellt man sich eine Lösung von 1,8 g Natrium in 800 ml absolutem Ethanol her. Bei 0 °C gibt man portionsweise 8,3 g 4-Amidino-benzoesäureamid-hydrochlorid zu. Die Suspension wird 10 Minuten bei 0 °C gerührt. Anschließend werden 8,7 g Ethoxymethylen-malonsäure-diethylester zugegeben. Man rührt 30 Minuten bei Raumtemperatur und erwärmt 2 Stunden unter Rückfluß. Nach weiteren 16 Stunden Rühren bei Raumtemperatur wird der Niederschlag abgenutscht und mit Ethanol gewaschen. Der Rückstand wird mit Wasser verrieben, erneut abgenutscht und getrocknet.
Ausbeute: 10,15 g (88 % der Theorie),
$R_f$-Wert: 0,78 (Kieselgel; Methylenchlorid/Methanol/konz. Ammoniaklösung = 2:1:0,25)

Beispiel XIV

1:1-Gemisch aus 5-Carboxy-4-chlor-2-(4-cyanophenyl)-pyrimidin und 4-Chlor-5-chlorformyl-2-(4-cyanophenyl)-pyrimidin

Eine Lösung von 12,6 g 2-(4-Carbamoyl-phenyl)-5-carboxy-(3H)-pyrimidin-4-on in 100 ml Phosphoroxychlorid wird 4 Stunden unter Rückfluß erwärmt. Man läßt abkühlen und gießt die Reaktionslösung portionsweise auf Wasser, wobei die Temperatur durch Zugabe von Eis zwischen 60-90 °C gehalten wird. Der Niederschlag wird abgenutscht und getrocknet. Man erhält 10,3 g eines 1:1-Gemisches aus Carbonsäure und Säurechlorid, welches als solches in Beispiel XV weiter umgesetzt wird.

Beispiel XV

4-Chlor-2-(4-cyanophenyl)-5-[4-[(methoxycarbonyl)-methyl]-piperidinocarbonyl]-pyrimidin

Eine Lösung von 7,0 g des 1:1-Gemisches aus 5-Carboxy-4-chlor-2-(4-cyanophenyl)-pyrimidin und 4-Chlor-5-chlorformyl-2-(4-cyanophenyl)-pyrimidin (aus Beispiel XIV) in 60 ml Thionylchlorid wird 3 Stunden unter Rückfluß erwärmt. Überschüssiges Thionylchlorid wird abgedampft, der Rückstand in trockenem Tetrahydrofuran gelöst und erneut einrotiert. Man löst in 200 ml trockenem Tetrahydrofuran und gibt 4,45 g Piperidin-4-essigsäure-methylester-hydrochlorid zu. Anschließend tropft man unter Kühlung im Eis/Wasser-Bad eine Lösung von 10,5 ml Triethylamin in 25 ml Tetrahydrofuran zu und rührt 16 Stunden bei Raumtemperatur. Die Suspension wird mit 1N Salzsäure angesäuert und die wässrige Phase mit Essigester extrahiert. Die organische Phase wird eingedampft und der Rückstand chromatographisch gereinigt.
Ausbeute: 5,95 g (65 % der Theorie),
$R_f$-Wert: 0,56 (Kieselgel; Methylenchlorid/Methanol = 15:1)

Beispiel XVI

2-(4-Cyanophenyl)-5-[4-[(methoxycarbonyl)-methyl]-piperidino-carbonyl]-(3H)-pyrimidin-4-on

Eine Lösung von 2,0 g 4-Chlor-2-(4-cyanophenyl)-5-[4-[(methoxycarbonyl)-methyl]-piperidinocarbonyl]-pyrimidin und 4,1 g Natriumacetat in 150 ml Eisessig wird 24 Stunden unter Rückfluß erwärmt. Der Eisessig wird abgedampft und der Rückstand mit 200 ml Wasser verrieben. Man nutscht den Niederschlag ab und reinigt den Feststoff chromatographisch über Kieselgel. Ausbeute: 0,85 g (45 % der Theorie),
$R_f$-Wert: 0,40 (Kieselgel; Methylenchlorid/Methanol = 15:1)

Beispiel XVII

2-(4-Cyanophenyl)-5-[4-[(methoxycarbonyl)-methyl]-piperidinocarbonyl]-pyrimidin

1,0 g 5%iges Palladium auf Calciumcarbonat in 100 ml trockenem Methanol wird vorhydriert. Es werden 1,5 g 4-Chlor-2-(4-cyanophenyl)-5-[4-[(methoxycarbonyl)-methyl]-piperidinocarbonyl]-pyrimidin und 0,28 g Calciumhydroxid zugegeben und 1,5 Stunden bei Raumtemperatur und Atmosphärendruck hydriert. Die unlöslichen Bestandteile werden abfiltriert, das Filtrat einrotiert und der Rückstand über Kieselgel chromatographiert.

Ausbeute: 650 mg (47 % der Theorie),
$R_f$-Wert: 0,46 (Kieselgel; Essigester/Cyclohexan = 2:1)
Analog wird folgende Verbindung erhalten:
(1) 3-(4-Cyanophenyl)-5-[[trans-4-(methoxycarbonyl)-cyclohexyl] -aminocarbonyl] -pyridazin
$R_f$-Wert: 0,34 (Kieselgel; Essigester/Cyclohexan = 2:1)

Beispiel XVIII

6-(4-Cyanophenyl)-3-[3-[(ethoxycarbonyl)-methyloxy]-piperidino]-pyridazin

Eine Suspension von 1,50 g 3-Chloro-6-(4-cyanophenyl)-pyridazin, 1,90 g 3-[(Ethoxycarbonyl)-methyloxy]-piperidin und 1,70 g Kaliumcarbonat in 4 ml Dimethylsulfoxid wird eine Stunde bei 130 ° C gerührt. Die Suspension wird in Wasser gegossen und die wässrige Phase dreimal mit Essigester extrahiert. Trocknen der Essigester-Phase über Natriumsulfat und Abdampfen des Lösungsmittels liefern in Öl, welches über Kieselgel chromatographiert wird.
Ausbeute: 1,70 g (68 % der Theorie) farbloses Öl,
$R_f$-Wert: 0,42 (Kieselgel; Cyclohexan/Essigester = 2:3)
Analog werden folgende Verbindungen erhalten:
(1) 6-(4-Cyanophenyl)-3-[[trans-4-(methoxycarbonyl)-cyclohexyl]-amino]-pyridazin
$R_f$-Wert: 0,20 (Kieselgel; Methylenchlorid/Methanol = 100:1)
(2) 6-(4-Cyanophenyl)-3-[[3-(methoxycarbonyl)-phenyl]-methylamino]-pyridazin
$R_f$-Wert: 0,48 (Kieselgel; Methylenchlorid/Methanol = 40:1)
(3) 6-(4-Cyanophenyl)-3-[4-[2-(methoxycarbonyl)-ethyl]-phenyloxy]-pyridazin
Es wird 2,5 Stunden auf 80-90 ° C erwärmt.
Schmelzpunkt: 164-167 ° C,
$R_f$-Wert: 0,19 (Kieselgel; Methylenchlorid/Methanol = 100:1)
(4) 6-(4-Cyanophenyl)-3-[4-[(methoxycarbonyl)-methyloxy]-phenyloxy]-pyridazin
Es wird 2,5 Stunden auf 80-90 ° C erwärmt. Das Rohprodukt wird mit Essigester verrieben und der Niederschlag abgesaugt und getrocknet.
$R_f$-Wert: 0,45 (Kieselgel; Methylenchlorid/Methanol = 20:1)
(5) 6-(4-Cyanophenyl)-3-[[N[trans-4-(methoxycarbonyl)-cyclohexyl]-N-methyl]-amino]-pyridazin
$R_f$-Wert: 0,21 (Kieselgel; Methylenchlorid/Methanol = 30:1)

Beispiel XIX

6-(4-Cyanophenyl)-3-[4-[2-(methoxycarbonyl)-ethyl]-phenylamino]-pyridazin

0,60 g 3-Chlor-6-(4-cyanophenyl)-pyridazin wird mit 1,70 g 4-[2-(methoxycarbonyl)-ethyl]-anilin 1,5 Stunden auf 140-150 ° C erhitzt. Die erkaltete Schmelze wird in Methylenchlorid gelöst und die organische Phase mit 1N Natronlauge extrahiert. Trocknen der organischen Phase über Natriumsulfat und Abdampfen des Lösungsmittels ergeben einen Feststoff, welcher über Kieselgel chromatographiert wird.
Ausbeute: 1,80 g (95 % der Theorie),
Schmelzpunkt: 212-217 ° C,
$R_f$-Wert: 0,34 (Kieselgel; Methylenchlorid/Methanol = 20:1)

Beispiel XX

6-(4-Cyanophenyl)-4-[[trans-4-(methoxycarbonyl)-cyclohexyl]aminocarbonyl]-3-morpholino-pyridazin

Eine Lösung von 1,0 g 3-Chlor-6-(4-cyanophenyl)-4-[[trans-4-(methoxycarbonyl)-cyclohexyl]-aminocarbonyl]-pyridazin in 10 ml Morpholin wird 2 Stunden zum Rückfluß erwärmt. Überschüssiges Morpholin wird abgedampft, der Rückstand mit 0,5 N Natronlauge versetzt und die wässrige Phase mit Methylenchlorid extrahiert. Die organische Phase wird über Natriumsulfat getrocknet und eingedampft. Man erhält 1,1 g Rohprodukt, welches über Kieselgel chromatographiert wird.
Ausbeute: 650 mg (49 % der Theorie),
Schmelzpunkt: 269-271 ° C
$R_f$-Wert: 0,29 (Kieselgel; Methylenchlorid/Methanol = 20:1)

Beispiel XXI

Cis- und trans-4-(N,N-Dibenzylamino)-cyclohexyl-carbonsäuremethylester

Eine Lösung von 68,0 g eines cis/trans-Gemisches des 4-Aminocyclohexyl-carbonsäure-methylesters, 154 g Benzylbromid und 156 g N-Ethyl-diisopropylamin in 300 ml Methanol werden 2 Stunden zum Rückfluß erwärmt. Die Reaktionslösung wird eingedampft. Zum Rückstand gibt man Wasser und anschließend soviel 1N Natronlauge, bis die Suspension alkalisch ist. Man extrahiert mehrmals mit Essigester, trocknet die organische Phase über Natriumsulfat und dampft das Lösungsmittel ab. Man erhält 130 g eines Öls, welches mit Cyclohexan/Essigester im Verhältnis 16:1 über Kieselgel chromatographiert wird.
Ausbeute: 54 g cis-4-(N,N-Dibenzylamino)-cyclohexyl-carbonsäure-methylester (46 % der Theorie),
Schmelzpunkt: 114-116 °C
$R_f$-Wert: 0,61 (Kieselgel; Cyclohexan/Essigester = 4:1)
Ausbeute: 25,5 g trans-4-(N,N-Dibenzylamino)-cyclohexyl-carbonsäure-methylester (22 % der Theorie),
Schmelzpunkt: 71-74 °C
$R_f$-Wert: 0,56 (Kieselgel; Cyclohexan/Essigester = 4:1)
Analog werden folgende Verbindungen erhalten:

(1) Cis- und trans-4-(N-Benzyl-methylamino)-cyclohexylcarbonsäure-methylester
Das Rohprodukt wird mit Cyclohexan/Essigester im Verhältnis 20:1 über Aluminiumoxid der Aktivitätsstufe III chromatographiert.
cis-4-(N-Benzyl-methylamino)-cyclohexyl-carbonsäure-methylester
$R_f$-Wert: 0,35 (Aluminiumoxid; Cyclohexan/Essigester = 10:1) trans-4-(N-Benzyl-methylamino)-cyclohexyl-carbonsäure-methylester
$R_f$-Wert: 0,27 (Aluminiumoxid; Cyclohexan/Essigester = 10:1)

Beispiel XXII

trans-4-Aminocyclohexyl-carbonsäure-methylester

Eine Suspension von 25 g trans-4-(N,N-Dibenzylamino)-cyclohexyl-carbonsäure-methylester und 5,0 g 10%iges Palladium auf Kohle in 500 ml Methanol wird eine Stunde bei 40 °C und einem Wasserstoffdruck von 3.6 bar hydriert. Der Katalysator wird abfiltriert und das Filtrat eingedampft.
Ausbeute: 11,0 g Öl (95 % der Theorie),
$R_f$-Wert: 0,69 (Kieselgel; Methylenchlorid/Methanol/konz. Ammoniaklösung = 4:1:0,25)
Analog werden folgende Verbindungen erhalten:

(1) cis-4-Aminocyclohexyl-carbonsäure-methylester
$R_f$-Wert: 0,69 (Kieselgel; Methylenchlorid/Methanol/konz. Ammoniaklösung = 4:1:0,25)
(2) trans-4-(Methylamino)-cyclohexyl-carbonsäure-methylester
$R_f$-Wert: 0,75 (Kieselgel; Methylenchlorid/Methanol/konz. Ammoniaklösung = 4:1:0,25)

Beispiel XXIII

6-(4-Cyanophenyl)-4-[[trans-4-(methoxycarbonyl)-cyclohexyl]-aminocarbonyl]-2-[(morpholinocarbonyl)-methyl]-(2H)-pyridazin-3-on

Eine Suspension aus 1,2 g 4-Carboxy-6-(4-cyanophenyl)-2-[(morpholinocarbonyl)-methyl]-(2H)-pyridazin-3-on, 1,29 g 2-[(1H)-Benzotriazol-1-yl]-1,1,3,3-tetramethyl-uroniumtetrafluorborat, 0,64 g trans-4-Amino-cyclohexancarbonsäure-methylester-hydrochlorid, 0,54 g 1-Hydroxy-(1H)-benzotriazolhydrat und 0,81 g N-Methylmorpholin in 100 ml Dimethylformamid wird 16 Stunden bei Raumtemperatur gerührt. Das Lösungsmittel wird bei einer Badtemperatur von 80 °C unter vermindertem Druck abgedampft und das verbleibende Öl chromatographiert.
Ausbeute: 1,38 g (83 % der Theorie),
Schmelzpunkt: 195-200 °C
$R_f$-Wert: 0,63 (Kieselgel; Methylenchlorid/Methanol = 9:1)

Herstellung der Endverbindungen

Beispiel 1

6-(4-Amidinophenyl)-4-[(4-carboxybutyl)-aminocarbonyl]-2-methyl-(2H)-pyridazin-3-on

Eine Lösung von 300 mg 6-(4-Amidinophenyl)-4-[[4-(methoxycarbonyl)-butyl]-aminocarbonyl]-2-methyl-(2H)-pyridazin-3-on und 125 mg Lithiumhydroxyd-monohydrat in einem Gemisch aus 20 ml Tetrahydrofuran und 16 ml Wasser wird 1,5 Stunden bei Raumtemperatur gerührt. Anschließend wird 1,0 g Ammoniumchlorid zugegeben und 20 Minuten gerührt. Überschüssiges Tetrahydrofuran wird im Vakuum abgedampft, der Niederschlag abgenutscht, mit Wasser und Aceton gewaschen und getrocknet. Ausbeute: 250 mg (91 % der Theorie),
Schmelzpunkt: über 250 °C,
Massenspektrum: 372 $(M+1)^+$
$R_f$-Wert: 0,24 (Kieselgel; Methylenchlorid/Methanol/konz. Ammoniaklösung = 2:1:0,25)
Analog werden folgende Verbindungen erhalten:

(1) 6-(4-Amidinophenyl)-4-[(3-carboxypropyl)-aminocarbonyl]-2-methyl-(2H)-pyridazin-3-on
Man verwendet 1N Natronlauge in Methanol.
Schmelzpunkt: ab 280 °C (Zers.),
Massenspektrum: 358 $(M+1)^+$
$R_f$-Wert: 0,26 (Kieselgel; Methylenchlorid/Methanol/konz. Ammoniaklösung = 2:1:0,25)

(2) 6-(4-Amidinophenyl)-4-[(2-carboxyethyl)-aminocarbonyl]-2-methyl-(2H)-pyridazin-3-on
Schmelzpunkt: ab 265 °C (Zers.),
Massenspektrum: 344 $(M+1)^+$
$R_f$-Wert: 0,19 (Kieselgel; Methylenchlorid/Methanol/konz. Ammoniaklösung = 2:1:0,25)

| | | | | | | |
|---|---|---|---|---|---|---|
| Ber.:<br>Gef.: | C | 50,66<br>50,78 | H | 5,58<br>5,63 | N | 18,64<br>18,66 |

(3) 6-(4-Amidinophenyl)-4-[(4-carboxybutyl)-aminocarbonyl]-2H-pyridazin-3-on
Massenspektrum: 358 $(M+1)^+$
$R_f$-Wert: 0,27 (Kieselgel; Methylenchlorid/Methanol/konz. Ammoniaklösung = 2:1:0,25)

(4) 6-(4-Amidinophenyl)-4-[(trans-4-carboxycyclohexyl)-aminocarbonyl]-2-methyl-(2H)-pyridazin-3-on
Schmelzpunkt: 263-266 °C,
Massenspektrum: 398 $(M+1)^+$
$R_f$-Wert: 0,16 (Kieselgel; Methylenchlorid/Methanol/konz. Ammoniaklösung = 2:1:0,25)

| | | | | | | |
|---|---|---|---|---|---|---|
| Ber. x 1 $H_2O$:<br>Gef.: | C | 57,82<br>57,99 | H | 6,07<br>6,08 | N | 16,86<br>16,92 |

(5) 6-(4-Amidinophenyl)-4-[(cis-4-carboxycyclohexyl)-aminocarbonyl]-2-methyl-(2H)-pyridazin-3-on
Schmelzpunkt: 266-269 °C,
Massenspektrum: 398 $(M+1)^+$
$R_f$-Wert: 0,15 (Kieselgel; Methylenchlorid/Methanol/konz. Ammoniaklösung = 2:1:0,25)

(6) 6-(4-Amidinophenyl)-4-[[4-(carboxymethyl)-phenyl]-aminocarbonyl]-2-methyl-(2H)-pyridazin-3-on
Man verwendet 1N Natronlauge und als Lösungsmittel ein Gemisch aus Methylenchlorid/Methanol/Tetrahydrofuran im Verhältnis 1:1:0,6.
Schmelzpunkt: ab 255 °C (Zers.)
Massenspektrum: 406 $(M+1)^+$

(7) 6-(4-Amidinophenyl)-3-[4-(2-carboxyethyl)-phenylamino]-pyridazin
Schmelzpunkt: über 260 °C,
$R_f$-Wert: 0,09 (Kieselgel; Methylenchlorid/Methanol/konz. Ammoniaklösung = 4:1:0,25)

| | | | | | | |
|---|---|---|---|---|---|---|
| Ber. x 0,5 $H_2O$:<br>Gef.: | C | 64,85<br>64,61 | H | 5,44<br>5,64 | N | 18,90<br>18,67 |

(8) 6-(4-Amidinophenyl)-3-[4-(2-carboxyethyl)-phenyloxy]-pyridazin
Das Rohprodukt wird chromatographisch gereinigt.
Massenspektrum: 363 $(M+1)^+$
$R_f$-Wert: 0,12 (Kieselgel; Methylenchlorid/Methanol/konz. Ammoniaklösung = 2:1:0,25)
(9) 6-(4-Amidinophenyl)-3-[(3-carboxyphenyl)-methylamino]-pyridazin
Schmelzpunkt: über 260 °C,
Massenspektrum: 348 $(M+1)^+$
$R_f$-Wert: 0,22 (Kieselgel; Methylenchlorid/Methanol/konz. Ammoniaklösung = 2:1:0,25)
(10) 6-(4-Amidinophenyl)-3-[3-[(carboxymethyl)-oxy]-piperidino]-pyridazin
Schmelzpunkt: ab 278 °C,
Massenspektrum: 356 $(M+1)^+$
$R_f$-Wert: 0,21 (Kieselgel; Methylenchlorid/Methanol/konz. Ammoniaklösung = 2:1:0,25)
(11) 6-(4-Amidinophenyl)-3-[(trans-4-carboxycyclohexyl)-amino]-pyridazin
Massenspektrum: 340 $(M+1)^+$
$R_f$-Wert: 0,14 (Kieselgel; Methylenchlorid/Methanol/konz. Ammoniaklösung = 2:1:0,25)
(12) 6-(4-Amidinophenyl)-3-[N-(trans-4-carboxycyclohexyl)-N-methylamino]-pyridazin
Schmelzpunkt: 308-310 °C,
Massenspektrum: 368 $(M+1)^+$
$R_f$-Wert: 0,25 (Kieselgel; Methylenchlorid/Methanol/konz. Ammoniaklösung = 2:1:0,25)
(13) 2-(4-Amidinophenyl)-5-[4-(carboxymethyl)-piperidinocarbonyl]-(3H)-pyrimidin-4-on
Massenspektrum: 383 $M^+$
$R_f$-Wert: 0,09 (Kieselgel; Methylenchlorid/Methanol/konz. Ammoniaklösung = 2:1:0,25)

| | | | | | | |
|---|---|---|---|---|---|---|
| Ber. x $H_2O$:<br>Gef.: | C | 56,85<br>57,06 | H | 5,78<br>5,59 | N | 17,45<br>17,15 |

(14) 2-(4-Amidinophenyl)-5-[4-(carboxymethyl)-piperidinocarbonyl]-4-methoxy-pyrimidin
Massenspektrum: 398 $(M+1)^+$
$R_f$-Wert: 0,38 (Kieselgel; Methylenchlorid/Methanol/konz. Ammoniaklösung = 2:1:0,25)
(15) 2-(4-Amidinophenyl)-5-[4-(carboxymethyl)-piperidinocarbonyl]-pyrimidin
Massenspektrum: 368 $(M+1)^+$
$R_f$-Wert: 0,20 (Kieselgel; Methylenchlorid/Methanol/konz. Ammoniaklösung = 2:1:0,25)
(16) 6-(4-Amidinophenyl)-4-[N-(trans-4-carboxycyclohexyl)-N-methyl-aminocarbonyl]-2-methyl-(2H)-pyridazin-3-on
Massenspektrum: 412 $(M+1)^+$
$R_f$-Wert: 0,11 (Kieselgel; Methylenchlorid/Methanol/konz. Ammoniaklösung = 4:1:0,25)
(17) 6-(4-Amidinophenyl)-2-(carbamoyl-methyl)-4-[(trans-4-carboxycyclohexyl)-aminocarbonyl]-(2H)-pyridazin-3-on
(18) 6-(4-Amidinophenyl)-2-benzyl-4-[(trans-4-carboxycyclohexyl)-aminocarbonyl]-(2H)-pyridazin-3-on
Massenspektrum: 474 $(M+H)^+$
$R_f$-Wert: 0,14 (Kieselgel; Methylenchlorid/Methanol/konz. Ammoniaklösung = 4:1:0,25)
(19) 6-(4-Amidinophenyl)-4-[(trans-4-carboxycyclohexyl)-aminocarbonyl]-(2H)-pyridazin-3-on
(20) 3-(4-Amidinophenyl)-5-[(trans-4-carboxycyclohexyl)-aminocarbonyl]-pyridazin
Schmelzpunkt: 292-296 °C,
Massenspektrum: 368 $(M+1)^+$
$R_f$-Wert: 0,07 (Kieselgel; Methylenchlorid/Methanol/konz. Ammoniaklösung = 4:1:0,25)
(21) 6-(4-Amidinophenyl)-4-[(trans-4-carboxycyclohexyl)-aminocarbonyl]-3-methoxy-pyridazin
Schmelzpunkt: Sinterung ab 270-280 °C,
Massenspektrum: 398 $(M+1)^+$
$R_f$-Wert: 0,26 (Kieselgel; Methylenchlorid/Methanol/konz. Ammoniaklösung = 2:1:0,25)
(22) 6-(4-Amidinophenyl)-4-[(trans-4-carboxycyclohexyl)-aminocarbonyl]-3-morpholino-pyridazin
Schmelzpunkt: 265-270 °C,
Massenspektrum: 453 $(M+1)^+$

$R_f$-Wert: 0,28 (Kieselgel; Methylenchlorid/Methanol/konz. Ammoniaklösung = 2:1:0,25)
(23) 6-(4-Amidinophenyl)-4-[(trans-4-carboxycyclohexyl)-aminocarbonyl]-3-ethoxy-pyridazin
(24) 6-(4-Amidinophenyl)-4-[(trans-4-carboxycyclohexyl)-aminocarbonyl]-3-thiomorpholino-pyridazin
(25) 6-(4-Amidinophenyl)-4-[(trans-4-carboxycyclohexyl)-aminocarbonyl]-3-dimethylamino-pyridazin
(26) 3-(4-Acetyl-piperazino)-6-(4-amidinophenyl)-4-[(trans-4-carboxycyclohexyl)-aminocarbonyl]-pyridazin
(27) 6-(4-Amidinophenyl)-4-[(trans-4-carboxycyclohexyl)-aminocarbonyl]-3-piperidino-pyridazin
(28) 6-(4-Amidinophenyl)-4-[(trans-4-carboxycyclohexyl)-aminocarbonyl]-3-pyrrolidino-pyridazin
(29) 6-(4-Amidinophenyl)-4-[(trans-4-carboxycyclohexyl)-aminocarbonyl]-2-[(N,N-dimethyl-aminocarbonyl)-methyl]-(2H)-pyridazin-3-on
(30) 6-(4-Amidinophenyl)-4-[(trans-4-carboxycyclohexyl)-aminocarbonyl]-2-[(morpholinocarbonyl)-methyl]-(2H)-pyridazin-3-on-hydrochlorid
Anstelle der Zugabe von Ammoniumchlorid wird mit 1N Salzsäure angesäuert.
Schmelzpunkt: Sinterung ab 245°C
Massenspektrum: 511 $(M+H)^+$
$R_f$-Wert: 0,16 (Kieselgel; Methylenchlorid/Methanol/konz. Ammoniaklösung = 2:1:0,25)
(31) 6-(4-Amidinophenyl)-4-[(trans-4-carboxycyclohexyl)-aminocarbonyl]-2-ethyl-(2H)-pyridazin-3-on
(32) 5-(4-Amidinophenyl)-3-[(trans-4-carboxycyclohexyl)-aminocarbonyl]-pyridin-2-on
(33) 5-(4-Amidinophenyl)-3-[(trans-4-carboxycyclohexyl)-aminocarbonyl]-1-methyl-pyridin-2-on
(34) 5-(4-Amidinophenyl)-3-[(trans-4-carboxycyclohexyl)-aminocarbonyl]-(1H)-pyrazin-2-on
(35) 6-(4-Amidinophenyl)-4-[(trans-4-carboxycyclohexyl)-aminocarbonyl]-2-phenyl-(2H)-pyridazin-3-on
(36) 6-(4-Amidino-2-methyl-phenyl)-4-[(trans-4-carboxycyclohexyl)-aminocarbonyl]-2-methyl-(2H)-pyridazin-3-on
(37) 6-(4-Amidino-2-fluor-phenyl)-4-[(trans-4-carboxycyclohexyl)-aminocarbonyl]-2-methyl-(2H)-pyridazin-3-on
(38) 3-(4-Amidinophenyl)-5-[(trans-4-carboxycyclohexyl)-aminocarbonyl]-(1H)-1,2,4-triazin-6-on
(39) 6-(4-Amidinophenyl)-4-[[[(carboxymethyl)-aminocarbonyl]-methyl]-aminocarbonyl]-2-methyl-(2H)-pyridazin-3-on
(40) 6-(4-Amidinophenyl)-4-[[[N-(carboxymethyl)-N-methylaminocarbonyl]-methyl]-aminocarbonyl]-2-methyl-(2H)-pyridazin-3-on
(41) 4-[[2-[N-Acetyl-N-(carboxymethyl)-amino]-ethyl]-aminocarbonyl]-6-(4-amidinophenyl)-2-methyl-(2H)-pyridazin-3-on
(42) 6-(4-Amidinophenyl)-4-[[2-[(carboxymethyl)-oxy]-ethyl]-aminocarbonyl]-2-methyl-(2H)-pyridazin-3-on
(43) 6-(4-Amidinophenyl)-4-[[2-[N-(carboxymethyl)-N-(methansulfonyl)-amino]-ethyl]-aminocarbonyl]-2-methyl-(2H)pyridazin-3-on
(44) 6-(4-Amidinophenyl)-4-[[2-[N-benzyl-N-(carboxymethyl)-amino]-ethyl]-aminocarbonyl]-2-methyl-(2H)-pyridazin-3-on
(45) 6-(4-Amidinophenyl)-4-[(3-carboxy-prop-2-enyl)-aminocarbonyl]-2-methyl-(2H)-pyridazin-3-on
(46) 5-(4-Amidinophenyl)-2-[4-(carboxymethyl)-piperidinocarbonyl]-pyrimidin
(47) 3-Amidino-6-[4-[4-(carboxymethyl)-piperidinocarbonyl]-phenyl]-pyridin
(48) 5-(4-Amidinophenyl)-2-[(3-carboxypropyl)-aminocarbonyl]-pyrimidin
(49) 5-(4-Amidinophenyl)-2-[N-(3-carboxymethyl)-N-methylaminocarbonyl]-pyrimidin
(50) 5-(4-Amidinophenyl)-2-[(4-carboxybutyl)-aminocarbonyl]-pyrimidin
(51) 2-[[2-[N-Acetyl-N-(carboxymethyl)-amino]-ethyl]-aminocarbonyl]-5-(4-amidinophenyl)-pyrimidin
(52) 5-(4-Amidinophenyl)-2-[4-(carboxymethyl)-piperidinocarbonyl]-pyrazin
(53) 2-(4-Amidinophenyl)-5-[4-(carboxymethyl)-piperidinocarbonyl]-3-methyl-(3H)-pyrimidin-4-on
(54) 3-Amidino-6-[4-[(trans-4-carboxycyclohexyl)-aminocarbonyl]-phenyl]-pyridin
(55) 5-(4-Amidino-3-fluorphenyl)-2-[4-(carboxymethyl)-piperidinocarbonyl]-pyrimidin
(56) 5-(4-Amidino-2-chlorphenyl)-2-[4-(carboxymethyl)-piperidinocarbonyl]-pyrimidin
(57) 2-(4-Amidino-2-methyl-phenyl)-5-[4-(carboxymethyl)-piperidinocarbonyl]-pyrimidin
(58) 5-(5-Amidinopyridin-2-yl)-2-[4-(carboxymethyl)-piperidinocarbonyl]-pyrimidin
(59) 5-(4-Amidinophenyl)-2-[4-(carboxymethyl)-piperidinocarbonyl]-pyridin
(60) 5-Amidino-2-[4-[4-(carboxymethyl)-piperidinocarbonyl]-phenyl]-pyrimidin
(61) 5-(4-Amidinophenyl)-2-[4-(carboxymethyl)-piperazinocarbonyl]-pyrimidin
(62) 5-(4-Amidinophenyl)-2-[4-(2-carboxyethyl)-piperazinocarbonyl]-pyrimidin
(63) 5-(4-Amidinophenyl)-2-[4-(1-carboxyethyl)-piperidinocarbonyl]-pyrimidin
(64) 5-(4-Amidinophenyl)-2-[4-(carboxymethyl)-3-oxy-piperazinocarbonyl]-pyrimidin
(65) 5-(4-Amidinophenyl)-2-[3-(carboxymethyl)-piperidinocarbonyl]-pyrimidin
(66) 6-(4-Amidinophenyl)-3-[3-[(carboxymethyl)-oxy]-pyrrolidino]-pyridazin

(67) 6-(4-Amidinophenyl)-3-[3-(2-carboxyethyl)-piperidino]-pyridazin
(68) 6-(4-Amidinophenyl)-3-[4-(2-carboxyethyl)-piperidino]-pyridazin
(69) 6-(4-Amidinophenyl)-3-[4-(2-carboxyethyl)-piperazino]-pyridazin
(70) 6-(4-Amidinophenyl)-3-[4-(carboxymethyl)-piperidino]-pyridazin
(71) 6-(4-Amidinophenyl)-3-[4-(carboxymethyl)-piperazino]-pyridazin
(72) 6-(4-Amidinophenyl)-3-[[4-(carboxymethyl)-cyclohexyl]-amino]-pyridazin
(73) 6-(4-Amidinophenyl)-3-[N-[4-(carboxymethyl)-cyclohexyl]-N-methyl-amino]-pyridazin
(74) 6-(4-Amidinophenyl)-3-[N-(4-carboxybutyl)-N-methylamino]-pyridazin
(75) 6-(4-Amidinophenyl)-3-[N-(5-carboxypentyl)-N-methyl-amino]-pyridazin
(76) 3-Amidino-6-[4-[(4-carboxybutyl)-oxy]-phenyl]-pyridin
(77) 2-Amidino-5-[4-[(5-carboxypentyl)-oxy]-phenyl]-pyrazin
(78) 5-(4-Amidinophenyl)-2-[trans-4-(carboxycyclohexyl)-amino]-pyrimidin
(79) 5-(4-Amidinophenyl)-2-[trans-4-(carboxycyclohexyl)-amino]-pyridin
(80) 5-Amidino-2-[4-[[(2-carboxyethyl)-aminocarbonyl]-methyloxy]-phenyl]-pyrimidin
(81) 5-Amidino-2-[4-[(4-carboxy-piperidinocarbonyl)-methyloxy]-phenyl]-1,3-thiazol
(82) 2-Amidino-5-[4-[[(carboxymethyl)-aminocarbonyl]-methyloxy]-phenyl]-pyrimidin
(83) 6-(4-Amidinophenyl)-3-amino-4-[N-(trans-4-carboxycyclohexyl)-N-methyl-aminocarbonyl]-pyridazin
(84) 6-(4-Amidinophenyl)-4-[(trans-4-carboxycyclohexyl)-aminocarbonyl]-2-[[(1-carboxy-2-phenyl-ethyl)-aminocarbonyl]-methyl]-(2H)-pyridazin-3-on
(85) 6-(4-Amidinophenyl)-4-[N-(trans-4-carboxycyclohexyl)-aminocarbonyl]-N-methyl-aminocarbonyl]-2-[[-(1-carboxyethyl)-aminocarbonyl]-methyl]-(2H)-pyridazin-3-on
(86) 2-[4-[N-[(3-Carboxyprop-1-yl)-carbonyl]-N-methyl-amino]-phenyl]-5-(N-methyl-amidino)-pyridin
(87) 5-(4-Amidinophenyl)-2-[[4-(carboxymethyl)-piperidino]-methyl]-pyrimidin
(88) 5-Amidino-2-[4-[(4-carboxybutyl)-aminosulfonyl]-phenyl]-pyrimidin
(89) 5-(4-Amidinophenyl)-2-[(4-(carboxymethyliden)-piperidino-carbonyl]-pyrimidin
(90) 2-(4-Amidinophenyl)-5-[4,4-bis-(Carboxymethyl)-piperidinocarbonyl]-pyrimidin
(91) 1-(4-Amidinophenyl)-5-[(trans-4-carboxycyclohexyl)-aminocarbonyl]-(1H)-pyridin-2-on
(92) 4-[4-[N-(n-Butyl)-amidino]-phenyl]-1-[[(2-carboxyethyl)-aminocarbonyl]-methyl]-(1H)-pyridin-2-on
(93) 2-[4-(Aminomethyl)-phenyl]-5-[4-(carboxymethyl)-piperidinocarbonyl]-(3H)-pyrimidin-4-on-hydrochlorid

Anstelle der Zugabe von Ammoniumchlorid wird mit 1N Salzsäure angesäuert.
Massenspektrum: 371 $(M+H)^+$
$R_f$-Wert: 0,01 (Kieselgel; Methylenchlorid/Methanol/konz. Ammoniaklösung = 2:1:0,25)

(94) 6-(4-Amidinophenyl)-4-[4-(carboxymethyl)-piperidinocarbonyl]-2-[(morpholinocarbonyl)-methyl]-(2H)-pyridazin-3-on
(95) 6-(4-Amidinophenyl)-4-[N-(trans-4-carboxycyclohexyl)-N-methyl-aminocarbonyl]-2-[-(morpholinocarbonyl)-methyl]-(2H)-pyridazin-3-on
(96) 2-(4-Amidinophenyl)-5-[(trans-4-carboxycyclohexyl)-aminocarbonyl]-4-methoxy-pyrimidin
(97) 2-(4-Amidinophenyl)-5-[N-(trans-4-carboxycyclohexyl)-N-methyl-aminocarbonyl]-pyrimidin
(98) 2-(4-Amidinophenyl)-5-[N-(trans-4-carboxycyclohexyl)-N-methyl-aminocarbonyl]-4-ethoxy-pyrimidin

## Beispiel 2

### 6-(4-Amidinophenyl)-4-[(trans-4-(methyoxycarbonyl)-cyclohexyl]-aminocarbonyl]-2-methyl-(2H)-pyridazin-3-on

2,40 g 6-(4-Cyanophenyl)-4-[[trans-4-(methoxycarbonyl)-cyclohexyl]-aminocarbonyl]-2-methyl-(2H)-pyridazin-3-on werden in 50 ml absolutem Methylenchlorid und 500 ml absolutem Methanol gelöst. Unter Kühlung im Eis/Wasser-Bad leitet man 2 Stunden trockenes HCl-Gas (vorgeschaltete Waschflasche mit konzentrierter Schwefelsäure) ein. Die Reaktionsösung wird bei Raumtemperatur gerührt und der Umsatz mittels Dünnschicht-Chromatographie kontrolliert. Nach vollständiger Umsetzung wird die Reaktionslösung bei einer Badtemperatur von 25-35 °C im Vakuum eingedampft, der Rückstand in 300 ml absolutem Methanol gelöst und die Lösung unter gutem Rühren mit 15,0 g Ammoniumcarbonat versetzt. Man rührt 16 Stunden bei Raumtemperatur, saugt den Niederschlag ab und wäscht mit Methanol und Wasser nach.
Ausbeute: 1,2 g (48 % der Theorie),
Schmelzpunkt: 198-205 °C
Massenspektrum: 412 $(M+1)^+$
Durch Einrotieren des Filtrats, Verreiben des Rückstandes mit Wasser und Abnutschen werden weitere 0,75

g Produkt mit Schmelzpunkt: 198-205 ° C erhalten.
$R_f$-Wert: 0,33 (Kieselgel; Methylenchlorid/Methanol/konz. Ammoniaklösung = 4:1:0,25)

| | | | | | | |
|---|---|---|---|---|---|---|
| Ber.: | C | 61,30 | H | 6,12 | N | 17,02 |
| Gef.: | | 61,05 | | 6,31 | | 16,91 |

Analog werden folgende Verbindungen erhalten:

(1) 6-(4-Amidinophenyl)-4-[[4-(methoxycarbonyl)-butyl]-aminocarbonyl]-2-methyl-(2H)-pyridazin-3-on
Als Ausgangsmaterial dient 6-(4-Cyanophenyl)-4-[(4-carboxybutyl)-aminocarbonyl]-2-methyl-(2H)-pyridazin-3-on
Massenspektrum: 386 $(M+1)^+$
$R_f$-Wert: 0,70 (Kieselgel; Methylenchlorid/Methanol/konz. Ammoniaklösung = 2:1:0,25)

(2) 6-(4-Amidinophenyl)-4-[[3-(methoxycarbonyl)-propyl]-aminocarbonyl]-2-methyl-(2H)-pyridazin-3-on
$R_f$-Wert: 0,31 (Kieselgel; Methylenchlorid/Methanol/konz. Ammoniaklösung = 2:1:0,25)

(3) 6-(4-Amidinophenyl)-4-[[2-(methoxycarbonyl)-ethyl]-aminocarbonyl]-2-methyl-(2H)-pyridazin-3-on
Als Ausgangsmaterial dient 6-(4-Cyanophenyl)-4-[[2-(ethoxycarbonyl)-ethyl]-aminocarbonyl]-2-methyl-(2H)-pyridazin-3-on
Schmelzpunkt: 182-185 ° C,
Massenspektrum: 358 $(M+1)^+$
$R_f$-Wert: 0,27 (Kieselgel; Methylenchlorid/Methanol/konz. Ammoniaklösung = 4:1:0,25)

(4) 6-(4-Amidinophenyl)-4-[[4-(methoxycarbonyl)-butyl]-aminocarbonyl]-(2H)-pyridazin-3-on
Als Ausgangsmaterial dient 6-(4-Cyanophenyl)-4-[(4-carboxybutyl)-aminocarbonyl]-(2H)-pyridazin-3-on. Das Rohprodukt wird chromatographisch gereinigt.
Massenspektrum: 372 $(M+1)^+$
$R_f$-Wert: 0,40 (Kieselgel; Methylenchlorid/Methanol/konz. Ammoniaklösung = 4:1:0,25)

(5) 6-(4-Amidinophenyl)-4-[[cis-4-(methoxycarbonyl)-cyclohexyl]-aminocarbonyl]-2-methyl-(2H)-pyridazin-3-on
Massenspektrum: 412 $(M+1)^+$
$R_f$-Wert: 0,24 (Kieselgel; Methylenchlorid/Methanol/konz. Ammoniaklösung = 4:1:0,25)

(6) 6-(4-Amidinophenyl)-4-[[4-[(methoxycarbonyl)-methyl]-phenyl]-aminocarbonyl]-2-methyl-(2H)-pyridazin-3-on-hydrochlorid Schmelzpunkt: ab 265 ° C (Zers.),
Massenspektrum: 402 $(M+1)^+$
$R_f$-Wert: 0,34 (Kieselgel; Methylenchlorid/Methanol/konz. Ammoniaklösung = 4:1:0,25)

(7) 6-(4-Amidinophenyl)-3-[4-[2-(methoxycarbonyl)-ethyl]-phenylamino]-pyridazin-hydrochlorid
Das Rohprodukt wird chromatographisch gereinigt.
Schmelzpunkt: über 200 ° C,
Massenspektrum: 376 $(M+1)^+$
$R_f$-Wert: 0,15 (Kieselgel; Methylenchlorid/Methanol/konz. Ammoniaklösung = 4:1:0,25)

| | | | | | | | | |
|---|---|---|---|---|---|---|---|---|
| Ber. x 1 HCl x 1 $H_2O$: | C | 58,67 | H | 5,63 | N | 16,29 | Cl | 8,25 |
| Gef.: | | 58,82 | | 5,74 | | 16,45 | | 8,04 |

(8) 6-(4-Amidinophenyl)-3-[4-[2-(methyloxycarbonyl)-ethyl]-phenyloxy]-pyridazin
Das Rohprodukt wird chromatographisch gereinigt.
Schmelzpunkt: 178-181 ° C,
Massenspektrum: 377 $(M+1)^+$
$R_f$-Wert: 0,40 (Kieselgel; Methylenchlorid/Methanol/konz. Ammoniaklösung = 4:1:0,25)

| | | | | | | |
|---|---|---|---|---|---|---|
| Ber.: | C | 67,01 | H | 5,36 | N | 14,88 |
| Gef.: | | 66,72 | | 5,31 | | 14,61 |

(9) 6-(4-Amidinophenyl)-3-[4-[(methoxycarbonyl)-methyloxy]-phenyloxy]-pyridazin und 6-(4-Amidinophenyl)-3-[4-(carboxymethyloxy)-phenyloxy]-pyridazin
Das Rohprodukt wird chromatographiert. Man erhält ein Gemisch aus dem Methylester und der freien Säure. Durch Verreiben des Gemisches mit Methylenchlorid/Methanol (1:1) und Absaugen des Niederschlags läßt sich die freie Säure anreichern.
$R_f$-Wert (Methylester): 0,58 (Kieselgel; Methylenchlorid/Methanol/konz. Ammoniaklösung = 2:1:0,25)
$R_f$-Wert (Säure): 0,44 (Kieselgel; Methylenchlorid/Methanol/konz. Ammoniaklösung = 2:1:0,25)
Massenspektrum (Säure): 365 $(M+1)^+$

(10) 6-(4-Amidinophenyl)-3-[[3-(methoxycarbonyl)-phenyl]-methylamino]-pyridazin-hydrochlorid
Die Reaktion wird in Methanol durchgeführt. Das Rohprodukt wird chromatographisch gereinigt.
Massenspektrum: 362 $(M+1)^+$
$R_f$-Wert: 0,34 (Kieselgel; Methylenchlorid/Methanol/konz. Ammoniaklösung = 4:1:0,25)

(11) 6-(4-Amidinophenyl)-3-[3-[(methoxycarbonyl)-methyl]-oxy]-piperidino]-pyridazin-hydrochlorid
Als Ausgangsprodukt wird 6-(4-Cyanophenyl)-3-[3-[(ethoxycarbonyl)-methyloxy]-piperidino]-pyridazin eingesetzt. Die Umsetzung erfolgt in Methanol.
$R_f$-Wert: 0,23 (Kieselgel; Methylenchlorid/Methanol/konz. Ammoniaklösung = 4:1:0,25)

(12) 6-(4-Amidinophenyl)-3-[[trans-4-(methoxycarbonyl)-cyclohexyl]-amino]-pyridazin
Die Reaktion wird in Methanol durchgeführt. Das Rohprodukt wird chromatographisch gereinigt.
Massenspektrum: 354 $(M+1)^+$
$R_f$-Wert: 0,28 (Kieselgel; Methylenchlorid/Methanol/konz. Ammoniaklösung = 4:1:0,25)

(13) 6-(4-Amidinophenyl)-3-[N-[trans-4-(methoxycarbonyl)-cyclohexyl]-N-methyl-amino]-pyridazin-hydrochlorid
Die Reaktion wird in Methanol durchgeführt. Das Rohprodukt wird chromatographisch gereinigt.
Schmelzpunkt: Sinterung ab 155 °C,
Massenspektrum: 368 $(M+1)^+$
$R_f$-Wert: 0,21 (Kieselgel; Methylenchlorid/Methanol/konz. Ammoniaklösung = 4:1:0,25)

(14) 2-(4-Amidinophenyl)-5-[4-[(methoxycarbonyl)-methyl]-piperidinocarbonyl]-(3H)-pyrimidin-4-on-hydrochlorid
Die Reaktion wird in Methanol durchgeführt. Das Rohprodukt wird chromatographisch gereinigt.
Massenspektrum: 398 $(M+1)^+$
$R_f$-Wert: 0,13 (Kieselgel; Methylenchlorid/Methanol/konz. Ammoniaklösung = 4:1:0,25)

(15) 2-(4-Amidinophenyl)-4-methoxy-5-[4-[(methoxycarbonyl)-methyl]-piperidinocarbonyl]-pyrimidin-hydrochlorid
Als Ausgangsmaterial dient 4-Chlor-2-(4-cyanophenyl)-5-[4-[(methoxycarbonyl)-methyl]-piperidinocarbonyl]-pyrimidin.
Die Reaktion wird in Methanol durchgeführt. Das Rohprodukt wird chromatographisch gereinigt.
Massenspektrum: 412 $(M+H)^+$
$R_f$-Wert: 0,07 (Kieselgel; Methylenchlorid/Methanol = 6:1)

(16) 2-(4-Amidinophenyl)-5-[4-[(methoxycarbonyl)-methyl]-piperidinocarbonyl]-pyrimidin-hydrochlorid
Die Reaktion wird in Methanol durchgeführt. Das Rohprodukt wird chromatographisch gereinigt.
Massenspektrum: 382 $(M+H)^+$
$R_f$-Wert: 0,29 (Kieselgel; Methylenchlorid/Methanol = 4:1)

(17) 6-(4-Amidinophenyl)-4-[N-[trans-4-(methoxycarbonyl)-cyclohexyl]-N-methyl-aminocarbonyl]-2-methyl-(2H)-pyridazin-3-on-hydrochlorid
Schmelzpunkt: Sinterung 200-215 °C,
Massenspektrum: 426 $(M+H)^+$
$R_f$-Wert: 0,52 (Kieselgel; Methylenchlorid/Methanol/konz. Ammoniaklösung = 4:1:0,25)

(18) 6-(4-Amidinophenyl)-2-(carbamoyl-methyl)-4-[[trans-4-(methoxycarbonyl)-cyclohexyl]-aminocarbonyl]-(2H)-pyridazin-3-on-hydrochlorid

(19) 6-(4-Amidinophenyl)-2-benzyl-4-[[trans-4-(methoxycarbonyl)-cyclohexyl]-aminocarbonyl]-(2H)-pyridazin-3-on Die Reaktion wird in Methanol durchgeführt.
Das Rohprodukt wird chromatographisch gereinigt.
Schmelzpunkt: 168-173 °C
Massenspektrum: 488 $(M+H)^+$
$R_f$-Wert: 0,27 (Kieselgel; Methylenchlorid/Methanol/konz. Ammoniaklösung = 4:1:0,25)

(20) 6-(4-Amidinophenyl)-4-[[trans-4-(methoxycarbonyl)-cyclohexyl]-aminocarbonyl]-(2H)-pyridazin-3-on-hydrochlorid

(21) 3-(4-Amidinophenyl)-5-[[trans-4-(methoxycarbonyl)-cyclohexyl]-aminocarbonyl]-pyridazin-hydrochlorid
Schmelzpunkt: 275-280 °C,
Massenspektrum: 382 $(M+1)^+$

$R_f$-Wert: 0,18 (Kieselgel; Methylenchlorid/Methanol/konz. Ammoniaklösung = 4:1:0,25)

(22) 6-(4-Amidinophenyl)-3-methoxy-4-[[trans-4-(methoxycarbonyl)-cyclohexyl]-aminocarbonyl]-pyridazin-hydrochlorid

Massenspektrum: 412 $(M+1)^+$

$R_f$-Wert: 0,29 (Kieselgel; Methylenchlorid/Methanol/konz. Ammoniaklösung = 4:1:0,25)

(23) 6-(4-Amidinophenyl)-4-[[trans-4-(methoxycarbonyl)-cyclohexyl]-aminocarbonyl]-3-morpholino-pyridazin-hydrochlorid

Schmelzpunkt: ab 290 ° C (Zers.),

Massenspektrum: 467 $(M+1)^+$

$R_f$-Wert: 0,16 (Kieselgel; Methylenchlorid/Methanol/konz. Ammoniaklösung = 4:1:0,25)

(24) 6-(4-Amidinophenyl)-2-ethoxy-4-[[trans-4-(ethoxycarbonyl)-cyclohexyl]-aminocarbonyl]-pyridazin-hydrochlorid

Die Umsetzung wird in absolutem Ethanol durchgeführt.

(25) 6-(4-Amidinophenyl)-4-[[trans-4-(methoxycarbonyl)-cyclohexyl]-aminocarbonyl]-3-thiomorpholino-pyridazin-hydrochlorid

(26) 6-(4-Amidinophenyl)-3-dimethylamino-4-[[trans-4-(methoxycarbonyl)-cyclohexyl]-aminocarbonyl]-pyridazin-hydrochlorid

(27) 6-(4-Acetyl-piperazino)-6-(4-amidinophenyl)-4-[[trans-4-(methoxycarbonyl)-cyclohexyl]-aminocarbonyl]-pyridazin-hydrochlorid

(28) 6-(4-Amidinophenyl)-4-[[trans-4-(methoxycarbonyl)-cyclohexyl]-aminocarbonyl]-3-piperidino-pyridazin-hydrochlorid

(29) 6-(4-Amidinophenyl)-4-[[trans-4-(methoxycarbonyl)-cyclohexyl]-aminocarbonyl]-3-pyrrolidino-pyridazin-hydrochlorid

(30) 6-(4-Amidinophenyl)-4-[[trans-4-(methoxycarbonyl)-cyclohexyl]-aminocarbonyl]-2-[(N,N-dimethyl-aminocarbonyl)-methyl-(2H)-pyridazin-3-on-hydrochlorid

(31) 6-(4-Amidinophenyl)-4-[[trans-4-(methoxycarbonyl)-cyclohexyl]-aminocarbonyl]-2-[-(morpholinocarbonyl)-methyl]-(2H)-pyridazin-3-on

Die Reaktion wird in Methanol durchgeführt. Das Rohprodukt wird chromatographisch gereinigt.

Massenspektrum: 524 $M^+$

$R_f$-Wert: 0,22 (Kieselgel; Methylenchlorid/Methanol/konz. Ammoniaklösung = 2:1:0,25)

(32) 6-(4-Amidinophenyl)-2-ethyl-4-[[trans-4-(methoxycarbonyl)-cyclohexyl]-aminocarbonyl]-(2H)-pyridazin-3-on-hydrochlorid

(33) 5-(4-Amidinophenyl)-3-[[trans-4-(methoxycarbonyl)-cyclohexyl]-aminocarbonyl]-(1H)-pyridin-2-on-hydrochlorid

(34) 5-(4-Amidinophenyl)-3-[[trans-4-(methoxycarbonyl)-cyclohexyl]-aminocarbonyl]-1-methyl-(1H)-pyridin-2-on-hydrochlorid

(35) 5-(4-Amidinophenyl)-3-[[trans-4-(methoxycarbonyl)-cyclohexyl]-aminocarbonyl]-(1H)-pyrazin-2-on-hydrochlorid

(36) 6-(4-Amidinophenyl)-4-[[trans-4-(methoxycarbonyl)-cyclohexyl]-aminocarbonyl]-2-phenyl-(2H)-pyridazin-3-on-hydrochlorid

(37) 6-(4-Amidino-2-methyl-phenyl)-4-[[trans-4-(methoxycarbonyl)-cyclohexyl]-aminocarbonyl]-2-methyl-(2H)-pyridazin-3-on-hydrochlorid

(38) 6-(4-Amidino-2-fluor-phenyl)-4-[[trans-4-(methoxycarbonyl)-cyclohexyl]-aminocarbonyl]-2-methyl-(2H)-pyridazin-3-on-hydrochlorid

(39) 3-(4-Amidino-5-[[trans-4-(methoxycarbonyl)-cyclohexyl]-aminocarbonyl]-(1H)-1,2,4-triazin-6-on-hydrochlorid

(40) 6-(4-Amidinophenyl)-4-[[[[(methoxycarbonyl)-methyl]-aminocarbonyl]-methyl]-aminocarbonyl]-2-methyl-(2H)-pyridazin-3-on-hydrochlorid

(41) 6-(4-Amidinophenyl)-4-[[[N-[(methoxycarbonyl)-methyl]-N-methyl-aminocarbonyl]-methyl]-aminocarbonyl]-2-methyl-(2H)-pyridazin-3-on-hydrochlorid

(42) 4-[[[N-Acetyl-N-[(methoxycarbonyl)-methyl]-aminocarbonyl]-methyl]-aminocarbonyl]-6-(4-amidinophenyl)-2-methyl-2H-pyridazin-2-on-hydrochlorid

(43) 6-(4-Amidinophenyl)-4-[[2-[[(methoxycarbonyl)-methyl]-oxy]-ethyl]-aminocarbonyl]-2-methyl]-(2H)-pyridazin-3-on-hydrochlorid

(44) 6-(4-Amidinophenyl)-4-[[2-[N-(methansulfonyl)-N-[(methoxycarbonyl)-methyl]-amino]-ethyl]-aminocarbonyl]-2-methyl-2H-pyridazin-3-on-hydrochlorid

(45) 6-(4-Amidinophenyl)-4-[[2-[N-benzoyl-N-[(methoxycarbonyl)-methyl]-amino]-ethyl]-aminocarbonyl]-2-methyl-(2H)-pyridazin-3-on-hydrochlorid

(46) 6-(4-Amidinophenyl)-2-methyl-4-[[3-(tetrazol-5-yl)-propyl]-aminocarbonyl]-(2H)-pyridazin-3-on-hydrochlorid
(47) 6-(4-Amidinophenyl)-2-methyl-4-[[4-(tetrazol-5-yl)-butyl]-aminocarbonyl]-(2H)-pyridazin-3-on-hydrochlorid
(48) 6-(4-Amidinophenyl)-2-methyl-4-[(3-phosphono-propyl)-aminocarbonyl]-(2H)-pyridazin-3-on-hydrochlorid
(49) 6-(4-Amidinophenyl)-2-methyl-4-[[4-(O-methyl-phosphono)-butyl]-aminocarbonyl]-(2H)-pyridazin-3-on-hydrochlorid
(50) 6-(4-Amidinophenyl)-2-methyl-4-[(4-sulfobutyl)-aminocarbonyl]-(2H)-pyridazin-3-on-hydrochlorid
(51) 5-(4-Amidinophenyl)-2-[4-[(methoxycarbonyl)-methyl]-piperidinocarbonyl)-pyrimidin-hydrochlorid
(52) 3-Amidino-6-[4-[4-[(methoxycarbonyl)-methyl]-piperidinocarbonyl]-phenyl]-pyridin-hydrochlorid
(53) 5-(4-Amidinophenyl)-2-[[3-(methoxycarbonyl)-propyl]-aminocarbonyl]-pyrimidin-hydrochlorid
(54) 5-(4-Amidinophenyl)-2-[N-[3-(methoxycarbonyl)-propyl]-N-methyl-aminocarbonyl]-pyrimidin-hydrochlorid
(55) 5-(4-Amidinophenyl)-2-[[4-(methoxycarbonyl)-butyl]-aminocarbonyl]-pyrimidin-hydrochlorid
(56) 2-[[2-[N-Acetyl-N-[(methoxycarbonyl)-methyl]-amino]-ethyl]-aminocarbonyl]-5-(4-amidinophenyl)-pyrimidin-hydrochlorid
(57) 5-(4-Amidinophenyl)-2-[4-[(methoxycarbonyl)-methyl]-piperidinocarbonyl]-pyrazin-hydrochlorid
(58) 2-(4-Amidinophenyl)-5-[4-[(methoxycarbonyl)-methyl]-piperidinocarbonyl]-3-methyl-(3H)-pyrimidin-4-on-hydrochlorid
(59) 3-Amidino-6-[4-[[trans-4-(methoxycarbonyl)-cyclohexyl]-aminocarbonyl]-phenyl]-pyridin-hydrochlorid
(60) 5-(4-Amidino-3-fluorphenyl)-2-[4-[(methoxycarbonyl)-methyl]-piperidinocarbonyl]-pyrimidin-hydrochlorid
(61) 5-(4-Amidino-2-chlorphenyl)-2-[4-[(methoxycarbonyl)-methyl]-piperidinocarbonyl]-pyrimidin-hydrochlorid
(62) 5-(4-Amidino-2-methyl-phenyl)-2-[4-[(methoxycarbonyl)-methyl]-piperidinocarbonyl]-pyrimidin-hydrochlorid
(63) 5-(5-Amidinopyridin-2-yl)-2-[4-[(methoxycarbonyl)-methyl]-piperidinocarbonyl]-pyrimidin-hydrochlorid
(64) 5-(4-Amidinophenyl)-2-[4-[(methoxycarbonyl)-methyl]-piperidinocarbonyl]-pyridin-hydrochlorid
(65) 5-Amidino-2-[4-[4-[(methoxycarbonyl)-methyl]-piperidinocarbonyl]-phenyl]-pyrimidin-hydrochlorid
(66) 5-(4-Amidinophenyl)-2-[4-[(methoxycarbonyl)-methyl]-piperazinocarbonyl]-pyrimidin-hydrochlorid
(67) 5-(4-Amidinophenyl)-2-[4-[2-(methoxycarbonyl)-ethyl]-piperazinocarbonyl]-pyrimidin-hydrochlorid
(68) 5-(4-Amidinophenyl)-2-[4-[1-(methoxycarbonyl)-ethyl]-piperidinocarbonyl]-pyrimidin-hydrochlorid
(69) 5-(4-Amidinophenyl)-2-[4-[(methoxycarbonyl)-methyl]-3-oxy-piperazinocarbonyl]-pyrimidin-hydrochlorid
(70) 5-(4-Amidinophenyl)-2-[3-[(methoxycarbonyl)-methyl]-piperidinocarbonyl]-pyrimidin-hydrochlorid
(71) 6-(4-Amidinophenyl)-3-[3-[[(methoxycarbonyl)-methyl]-oxy]-pyrrolidino]-pyridazin-hydrochlorid
(72) 6-(4-Amidinophenyl)-3-[3-[2-(methoxycarbonyl)-ethyl]-piperidino]-pyridazin-hydrochlorid
(73) 6-(4-Amidinophenyl)-3-[4-[2-(methoxycarbonyl)-ethyl]-piperidino]-pyridazin-hydrochlorid
(74) 6-(4-Amidinophenyl)-3-[4-[2-(methoxycarbonyl)-ethyl]-piperazino]-pyridazin-hydrochlorid
(75) 6-(4-Amidinophenyl)-3-[4-[(methoxycarbonyl)-methyl]-piperidino]-pyridazin-hydrochlorid
(76) 6-(4-Amidinophenyl)-3-[4-[(methoxycarbonyl)-methyl]-piperazino]-pyridazin-hydrochlorid
(77) 6-(4-Amidinophenyl)-3-[[4-[(methoxycarbonyl)-methyl]-cyclohexyl]-amino]-pyridazin-hydrochlorid
(78) 6-(4-Amidinophenyl)-3-[N-[4-[(methoxycarbonyl)-methyl]-cyclohexyl]-N-methyl-amino]-pyridazin-hydrochlorid
(79) 6-(4-Amidinophenyl)-3-[N-[4-[(methoxycarbonyl)-butyl]-N-methyl-amino]-pyridazin-hydrochlorid
(80) 6-(4-Amidinophenyl)-3-[N-[5-(methoxycarbonyl)-pentyl]-N-methyl-amino]-pyridazin-hydrochlorid
(81) 3-Amidino-5-[4-[[4-(methoxycarbonyl)-butyl]-oxy]-phenyl]-pyridin-hydrochlorid
(82) 2-Amidino-5-[4-[[5-(methoxycarbonyl)-pentyl]-oxy]-phenyl]-pyrazin-hydrochlorid
(83) 5-(4-Amidinophenyl)-2-[[trans-4-(methoxycarbonyl)-cyclohexyl]-amino]-pyrimidin-hydrochlorid
(84) 5-(4-Amidinophenyl)-2-[[trans-4-(methoxycarbonyl)-cyclohexyl]-amino]-pyridin-hydrochlorid
(85) 5-Amidino-2-[4-[[[2-(methoxycarbonyl)-ethyl]-aminocarbonyl]-methyloxy]-phenyl]-pyrimidin-hydrochlorid
(86) 5-Amidino-2-[4-[[4-(methoxycarbonyl)-piperidinocarbonyl]-methyloxy]-phenyl]-1,3-thiazol-hydrochlorid
(87) 2-Amidino-5-[4-[[[(methoxycarbonyl)-methyl]-aminocarbonyl]-methyloxy]-phenyl]-pyrimidin-hydrochlorid

(88) 6-(4-Amidinophenyl)-4-[[3-(methoxycarbonyl)-prop-2-en-yl]-aminocarbonyl]-2-methyl-(2H)-pyridazin-3-on-hydrochlorid

(89) 6-(4-Amidinophenyl)-3-amino-4-[N-[trans-4-(methoxycarbonyl)-cyclohexyl]-N-methyl-aminocarbonyl]-pyridazin-hydrochlorid

(90) 6-(4-Amidinophenyl)-4-[[trans-4-(methoxycarbonyl)-cyclohexyl]-aminocarbonyl]-2-[[[1-(methoxycarbonyl)-2-phenylethyl]-aminocarbonyl]-methyl]-(2H)-pyridazin-3-on-hydrochlorid

(91) 6-(4-Amidinophenyl)-4-[N-[trans-4-(methoxycarbonyl)-cyclohexyl]-N-methyl-aminocarbonyl]-2-[[[1-(methoxycarbonyl)-ethyl]-aminocarbonyl]-methyl]-(2H)-pyridazin-3-on-hydrochlorid

(92) 2-[4-[N-[[3-(Methoxycarbonyl)-propyl]-carbonyl]-N-methyl-amino]-phenyl]-5-(N-methyl-amidino)-pyridin-hydrochlorid Der Iminoester wird in absolutem Methanol gelöst und mit einem 20-fachen Überschuß einer methanolischen Methylaminlösung umgesetzt.

(93) 5-(4-Amidinophenyl)-2-[[4-[(methoxycarbonyl)-methyl]-piperidino]-methyl]-pyrimidin-hydrochlorid

(94) 5-Amidino-2-[4-[[4-(methoxycarbonyl)-butyl]-aminosulfonyl]-phenyl]-pyrimidin-hydrochlorid

(95) 5-(4-Amidinophenyl)-2-[4-[(methoxycarbonyl)-methyliden]-piperidinocarbonyl]-pyrimidin-hydrochlorid

(96) 2-(4-Amidinophenyl)-5-[4,4-bis-[(methoxycarbonyl)-methyl]-piperidinocarbonyl]-pyrimidin-hydrochlorid

(97) 1-(4-Amidinophenyl)-5-[[trans-4-(methoxycarbonyl)-cyclohexyl]-aminocarbonyl]-(1H)-pyridin-2-on-hydrochlorid

(98) 4-[4-[N-(n-Butyl)-amidino]-phenyl]-1-[[[2-(methoxycarbonyl)-ethyl]-aminocarbonyl]-methyl]-(1H)-pyridin-2-on-hydrochlorid

Der Iminoester wird in absolutem Methanol aufgenommen und mit einem 20-fachen Überschuß einer methanolischen n-Butylaminlösung umgesetzt.

(99) 6-(4-Amidinophenyl)-4-[4-[(methoxycarbonyl)-methyl]-piperidinocarbonyl]-2-[(morpholinocarbonyl)-methyl]-(2H)-pyridazin-3-on

(100) 6-(4-Amidinphenyl)-4-[N-[trans-4-(methoxycarbonyl)-cyclohexyl]-N-methyl-aminocarbonyl]-2-[-(morpholinocarbonyl)-methyl]-(2H)-pyridazin-3-on

(101) 2-(4-Amidinophenyl)-4-methoxy-5-[[trans-4-(methoxycarbonyl)-cyclohexyl]-aminocarbonyl]-pyrimidin

(102) 2-(4-Amidinophenyl)-5-[N-[trans-4-(methoxycarbonyl)-cyclohexyl]-N-methyl-aminocarbonyl]-pyrimidin

(103) 2-(4-Amidinophenyl)-4-ethoxy-5-[N-[trans-4-(ethoxycarbonyl)-cyclohexyl]-N-methyl-aminocarbonyl]-pyrimidin

Als Ausgangsmaterial dient 4-Chlor-2-(4-cyanophenyl)-5-[N-[trans-4-(methoxycarbonyl)-cyclohexyl]-N-methyl-aminocarbonyl]-pyrimidin. Die Umsetzung wird in Ethanol durchgeführt.

## Beispiel 3

### 6-[4-[N-(Methoxycarbonyl)-amidino]-phenyl]-4-[[trans-4-(methoxycarbonyl)-cyclohexyl]-aminocarbonyl]-2-methyl-(2H)-pyridazin-3-on

Zu einer Lösung von 300 mg 6-(4-Amidinophenyl)-4-[[trans-4-(methoxycarbonyl)-cyclohexyl]-aminocarbonyl]-2-methyl-(2H)-pyridazin-3-on in einem Gemisch aus 10 ml Methylenchlorid und 10 ml Methanol gibt man 95 mg Chlorameisensäure-methylester. Durch Zutropfen von 1N Natronlauge wird der pH-Wert der Lösung zwischen pH = 8.5-9.0 gehalten. Die Umsetzung wird mittels Dünnschichtchromatographie kontrolliert. Nach 1,5 Stunden werden weitere 0,1 ml Chlorameisensäure-methylester hinzugegeben. Nach 3 Stunden wird das organische Lösungsmittel abgedampft, die verbleibende wässrige Phase mit Wasser verdünnt und dreimal mit Methylenchlorid extrahiert. Nach dem Trocknen der organischen Phase über Natriumsulfat wird das Lösungsmittel abgedampft und der verbleibende Feststoff über Kieselgel chromatographiert.

Ausbeute: 290 mg (85 % der Theorie),

Massenspektrum: 470 $(M+1)^+$

$R_f$-Wert: 0,58 (Kieselgel; Methylenchlorid/Methanol = 9:1)

Analog werden folgende Verbindungen erhalten:

(1) 6-[4-[N-(Ethoxycarbonyl)-amidino]-phenyl]-4-[[trans-4-(methoxycarbonyl)-cyclohexyl]-aminocarbonyl]-2-methyl-(2H)-pyridazin-3-on

Es wird Chlorameisensäure-ethylester eingesetzt.

Massenspektrum: 483 $M^+$

$R_f$-Wert: 0,54 (Kieselgel; Methylenchlorid/Methanol = 9:1)

(2) 6-[4-[N-(Methoxycarbonyl)-amidino]-phenyl]-4-[[cis-4-(methoxycarbonyl)-cyclohexyl]-aminocarbonyl]-2-methyl-(2H)-pyridazin-3-on
Massenspektrum: 469 $M^+$
$R_f$-Wert: 0,73 (Kieselgel; Methylenchlorid/Methanol = 9:1)
(3) 4-Methoxy-2-[4-[N-(methoxycarbonyl)-amidino]-phenyl]-5-[4-[(methoxycarbonyl)-methyl]-piperidinocarbonyl]-pyrimidin
Schmelzpunkt: 193-195 ° C,
Massenspektrum: 469 $M^+$
$R_f$-Wert: 0,48 (Kieselgel; Methylenchlorid/Methanol = 9:1)
(4) 5-[4-[N-(Methoxycarbonyl)-amidino]-phenyl]-2-[4-[(methoxycarbonyl)-methyl]-piperidinocarbonyl]-pyrimidin
(5) 4-[N-[trans-4-(Benzyloxycarbonyl)-cyclohexyl]-N-methylaminocarbonyl]-6-[4-[N-(methoxycarbonyl)-amidino]-phenyl]-2-methyl-(2H)-pyridazin-3-on
Als Ausgangsverbindung wird der Benzylester eingesetzt.
(6) 6-[4-[N-(Methoxycarbonyl)-amidino]-phenyl]-4-[[trans-4-(methoxycarbonyl)-cyclohexyl]-aminocarbonyl]-2-[(morpholinocarbonyl)-methyl]-(2H)-pyridazin-3-on
(7) 6-[4-[[(Acetyloxymethyl)-oxycarbonyl]-amidino]-phenyl]-4-[[trans-4-(methoxycarbonyl)-cyclohexyl]-aminocarbonyl]-2-[(morpholinocarbonyl)-methyl]-(2H)-pyridazin-3-on
Es wird Kohlensäure-acetyloxymethyl-(4-nitrophenyl)-ester und N-Ethyl-diisopropylamin eingesetzt.
(8) 6-[4-[[(1-Acetyloxy-ethyl)-oxycarbonyl]-amidino]-phenyl]-4-[[trans-4-(ethoxycarbonyl)-cyclohexyl]-aminocarbonyl]-2-[(morpholinocarbonyl)-methyl]-(2H)-pyridazin-3-on
Es wird Kohlensäure-(1-acetyloxy-ethyl)-(4-nitrophenyl)-ester und N-Ethyl-diisopropylamin eingesetzt.
(9) 2-[4-[N-(Methoxycarbonyl)-amidino]-phenyl]-5-[N-[trans-4-(methoxycarbonyl)-cyclohexyl]-N-methyl-aminocarbonyl]-pyrimidin
(10) 2-[4-[(Diethylphosphono)-amidino]-phenyl]-5-[N-[trans-4-(methoxycarbonyl)-cyclohexyl]- N-methyl-aminocarbonyl]-pyrimidin
Die Umsetzung erfolgt mit Phosphorsäure-diethylester-chlorid in Tetrahydrofuran/Wasser unter Zutropfen von Natronlauge und anschließende chromatographische Reinigung.

## Beispiel 4

### 6-[4-(Aminomethyl)-phenyl]-4-[(trans-4-carboxycyclohexyl)-aminocarbonyl]-2-methyl-(2H)-pyridazin-3-on-hydrochlorid

Eine Suspension von 1,50 g 4-[(trans-4-Carboxycyclohexyl)-aminocarbonyl]-6-(4-cyanophenyl)-2-methyl-(2H)-pyridazin-3-on und 0,5 g Raney-Nickel in 500 ml konzentriertem ammoniakalischem Methanol wird 4,5 Stunden bei Raumtemperatur und einem Wasserstoffdruck von 5 bar hydriert. Der Katalysator wird abfiltriert und das Filtrat am Rotationsverdampfer eingedampft. Der verbleibende Feststoff wird über Kieselgel chromatographiert. Man erhält 1,0 g (66 % der Theorie) Produkt.

Zur Herstellung des Hydrochlorids werden 310 mg des Produkts in 150 ml Tetrahydrofuran suspendiert. Man tropft etherische Salzsäure zu und anschließend soviel Wasser bis eine Lösung entsteht. Die organischen Lösungsmittel werden abgedampft. Der Niederschlag wird abgenutscht, mit wenig Wasser gewaschen und bei 80 ° C getrocknet.
Ausbeute: 290 mg (56 % der Theorie),
Schmelzpunkt: Sinterung ab 287 ° C,
Massenspektrum: 384 $M^+$
$R_f$-Wert: 0,57 (Kieselgel; Methylenchlorid/Methanol/konz. Ammoniaklösung = 4:1:0,25)

| | | | | | | | | |
|---|---|---|---|---|---|---|---|---|
| Ber. x HCL x $H_2O$: | C | 54,73 | H | 6,20 | N | 12,76 | Cl | 8,08 |
| Gef.: | | 54,67 | | 6,06 | | 12,66 | | 7,96 |

Analog werden folgende Verbindungen erhalten:
(1) 6-[4-(Aminomethyl)-phenyl]-2-benzyl-4-[(trans-4-carboxycyclohexyl)-aminocarbonyl]-(2H)-pyridazin-3-on-hydrochlorid Massenspektrum: 460 $M^+$
$R_f$-Wert: 0,62 (Kieselgel; Methylenchlorid/Methanol/konz. Ammoniaklösung = 2:1:0,25)
(2) 5-Aminomethyl-2-[4-[4-(carboxymethyl)-piperidinocarbonyl]-phenyl]-pyridin

Beispiel 5

6-[4-(Aminomethyl)-phenyl]-4-[[trans-4-(methoxycarbonyl)-cyclohexyl]-aminocarbonyl]-2-methyl-(2H)-pyridazin-3-on-hydro-chlorid

Eine Lösung von 780 mg 6-[4-(Aminomethyl)-phenyl]-4-[(trans-4-carboxycyclohexyl)-aminocarbonyl]-2-methyl-(2H)-pyridazin-2-on in einem Gemisch aus 400 ml absolutem Methanol und 50 ml etherischer Salzsäure wird 16 Stunden bei Raumtemperatur gerührt. Das Lösungsmittel wird abgedampft und der Rückstand über Kieselgel chromatographiert.
Ausbeute: 440 mg (50 % der Theorie),
Schmelzpunkt: 175-178 ° C,
Massenspektrum: 398 $M^+$
$R_f$-Wert: 0,28 (Kieselgel; Methylenchlorid/Methanol = 9:1)
Analog werden folgende Verbindungen erhalten:

(1) 6-[4-(Aminomethyl)-phenyl]-2-benzyl-4-[[trans-4-(methoxycarbonyl)-cyclohexyl]-aminocarbonyl]-(2H)-pyridazin-3-on-hydrochlorid
Massenspektrum: 474 $M^+$
$R_f$-Wert: 0,64 (Kieselgel; Methylenchlorid/Methanol/konz. Ammoniaklösung = 8:1:0,1)
(2) 5-Aminomethyl-2-[4-[4-[(methoxycarbonyl)-methyl]-piperidinocarbonyl]-phenyl]-pyridin-hydrochlorid

Beispiel 6

2-[4-[N-(Benzyloxycarbonyl)-amidino]-phenyl]-5-[4-[(benzyloxycarbonyl)-methyl]-4-hydroxy-piperidinocarbonyl]-pyrimidin

Hergestellt durch die Umsetzung von 2-[4-[N-(Benzyloxycarbonyl)-amidino]-phenyl]-5-carboxy-pyrimidin mit 1,2 Equivalenten N,N-Carbonyl-diimidazol in Dimethylformamid bei 5 ° C und nachfolgender Zugabe von 1,2 Equivalenten 4-[(Benzyloxycarbonyl)-methyl]-4-hydroxy-piperidin-hydrochlorid und 1,2 Equivalenten N-Methylmorpholin bei Raumtemperatur.

Beispiel 7

2-(4-Amidinophenyl)-5-[4-(carboxymethyl)-4-hydroxy-piperidinocarbonyl]-pyrimidin

Hergestellt durch Hydrierung von 2-[4-[N-(Benzyloxycarbonyl)-amidino]-phenyl]-5-[4-[-(benzyloxycarbonyl)-methyl]-4-hydroxy-piperidinocarbonyl]-pyrimidin in Methanol in Gegenwart von 10%igem Palladium auf Kohle bei Raumtemperatur und einem Wassezstoffdruck von 5 bar.
Analog wird folgende Verbindungen erhalten:

(1) 4-[N-(trans-4-carboxy-cyclohexyl)-N-methyl-aminocarbonyl]-6-[4-[N-(methoxycarbonyl)-amidino]-phenyl]-2-methyl-2H-pyridazin-3-on

Beispiel 8

2-(4-Amidinophenyl)-5-[4-[[(pyridin-3-ylmethyl)-oxycarbonyl]-methyl]-piperidinocarbonyl]-pyrimidin-methansulfonat

Man verestert 2-(4-Amidinophenyl]-5-[4-(carboxymethyl)-piperidinocarbonyl]-pyrimidin mit einem 12-fachen Überschuß an 3-Hydroxymethyl-pyridin und einem 15-fachen Überschuß an Methansulfonsäure in Dimethylformamid.

Analog wird folgende Verbindungen erhalten:

(1) 6-(4-Amidinophenyl)-4-[N-[trans-4-(benzyloxycarbonyl)-cyclohexyl]-N-methyl-aminocarbonyl]-2-methyl-(2H)-pyridazin-3-on-toluolsulfonat
Man arbeitet in Benzylalkohol als Lösungsmittel unter Verwendung von 2 Equivalenten Toluolsulfonsäure.

Beispiel 9

6-(4-Amidinophenyl)-2-benzyl-4-[[trans-4-(isopropoxycarbonyl)-cyclohexyl]-aminocarbonyl]-(2H)-pyridazin-3-on

Eine Lösung von 130 mg 6-(4-Aminophenyl)-2-benzyl-4-[(trans-4-(carboxycyclohexyl)-aminocarbonyl]-(2H)-pyridazin-3-on in 30 ml mit Chlorwasserstoff-gesättigtem Isopropanol wird 30 Stunden bei Raumtemperatur gerührt. Das Lösungsmittel wird unter vermindertem Druck eingedampft und der Rückstand über Kieselgel chromatographiert.

Ausbeute: 40 mg (20 % der Theorie),
Massenspektrum: 516 $(M+H)^+$
$R_f$-Wert: 0,12 (Kieselgel; Methylenchlorid/Isopropanol/konz. Ammoniaklösung = 4:1:0,25)
Analog werden folgende Verbindungen erhalten:

(1) 6-(4-Amidinophenyl)-4-[[trans-4-(cyclohexyloxycarbonyl)-cyclohexyl]-aminocarbonyl]-2-[-(morpholinocarbonyl)-methyl]-(2H)-pyridazin-3-on
Die Umsetzung wird in einem mit Chlorowasserstoff-gesättigtem Gemisch aus Cyclohexanol/Methylenchlorid durchgeführt.

(2) 6-(4-Amidinophenyl)-4-[[trans-4-(cyclohexylmethyloxycarbonyl)-cyclohexyl]-aminocarbonyl]-2-[-(morpholinocarbonyl)-methyl]-(2H)-pyridazin-3-on
Die Umsetzung wird in einem mit Chlorwasserstoff-gesättigtem Gemisch aus Cyclohexylmethanol/Methylenchlorid durchgeführt.

(3) 6-(4-Amidinophenyl)-4-[[trans-4-(ethoxycarbonyl)-cyclohexyl]-aminocarbonyl]-2-[(morpholinocarbonyl)-methyl]-(2H)-pyridazin-3-on
Die Umsetzung wird in Chlorwasserstoff-gesättigtem Ethanol durchgeführt.

(4) 2-(4-Amidinophenyl)-5-[N-methyl-N-[trans-4-(isopropoxycarbonyl)-cyclohexyl]-aminocarbonyl]-pyrimidin

(5) 2-(4-Amidinophenyl)-5-[N-[trans-4-(cyclohexyloxycarbonyl)-cyclohexyl]-N-methyl-aminocarbonyl]-pyrimidin
Die Umsetzung wird in einem mit Chlorwasserstoff-gesättigtem Gemisch aus Cyclohexanol/Methylenchlorid durchgeführt.

(6) 2-(4-Amidinophenyl)-5-[N-[trans-4-(cyclopentylmethoxycarbonyl)-cyclohexyl]-N-methyl-aminocarbonyl]-pyrimidin
Die Umsetzung wird in einem mit Chlorwasserstoff-gesättigtem Gemisch aus Cyclopentylmethanol/Methylenchlorid durchgeführt.

Beispiel 10

2-[4-(Aminomethyl)-phenyl]-5-[4-[(methoxycarbonyl)-methyl]-piperidinocarbonyl]-(3H)-pyrimidin-4-on-hydrochlorid

Eine Suspension von 670 mg 2-(4-Cyanophenyl)-5-[4-[(methoxycarbonyl)-methyl]-piperidinocarbonyl]-(3H)-pyrimidin-4-on und 100 mg 10 % Palladium auf Kohle in 125 ml Methanol und 20 ml etherische Salzsäure werden 3 Stunden bei Raumtemperatur und einem Wasserstoffdruck von 3 bar hydriert. Der Katalysator wird abfiltriert und das Filtrat unter vermindertem Druck eingedampft. Der verbleibende Feststoff wird mit Ether verrieben und abgenutscht.

Ausbeute: 740 mg (100 % der Theorie),
Schmelzpunkt: 205 °C (Zers.)
$R_f$-Wert: 0,18 (Kieselgel; Methylenchlorid/Methanol/konz. Ammoniaklösung = 4:1:0,25)

Beispiel 11

Trockenampulle mit 2,5 mg Wirkstoff pro 1 ml

| Zusammensetzung: | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 12

Trockenampulle mit 35 mg Wirkstoff pro 2 ml

| Zusammensetzung: | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 13

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 14

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 15

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 16

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Heterobiarylderivate der allgemeinen Formel

$R_1NH - X_1 - X_2 - X_3 - Y_1 - Y_2 - Y_3 - Y_4 - E$ ,(I)

mit Ausnahme von 2-Guanidino-4-[3-[3-(methoxycarbonyl)-propyl]-phenyl]-1,3-thiazol und 2-Guanidino-4-[6-[(methoxycarbonyl)-methyl]-pyrid-2-yl]-1,3-thiazol, in der $R_1$ ein Wasserstoffatom, eine Alkyl-, Hydroxy-, Amino-, Alkoxycarbonyl-, Aralkoxycarbonyl-, Aryloxycarbonyl-, Alkylcarbonyl- oder Arylcarbonylgruppe, in denen der Alkyl-und Alkoxyteil jeweils 1 bis 4 Kohlenstoffatome enthalten kann, eine Alkenyloxycarbonylgruppe mit insgesamt 4 bis 6 Kohlenstoffatomen, eine Phosphono-, O-Alkylphosphono-, Dialkylphosphoryl- oder R'-CO-O-(R''CH)-O-CO-Gruppe, wobei

R' eine Alkylgruppe mit 1 bis 5 Kohlenstoffatomen, eine Cycloalkyl-, Cycloalkylalkyl-, Aryl- oder Aralkylgruppe und

R'' ein Wasserstoffatom, eine Alkylgruppe mit 1 bis 5 Kohlenstoffatomen, eine Cycloalkyl- oder Arylalkylgruppe darstellen,

$X_1$ eine Alkylengruppe mit 1 bis 3 Kohlenstoffatomen, eine -C( = NH)-, -C( = NH)-NH- oder -C( = NH)-NH-CO-Gruppe, wobei jeweils das Kohlenstoffatom der -C( = NH)-Gruppe der vorstehend erwähnten Reste mit dem Stickstoffatom des $R_1NH$-Restes verknüpft ist,

$X_2$ eine Phenylen-, Pyridinylen-, Pyrazinylen-, Pyrimidinylen- oder Pyridazinylengruppe, die jeweils im Kohlenstoffgerüst durch Fluor-, Chlor-, Brom- oder Jodatome, durch Alkyl-, Aralkyl-, Aryl-, Pyridylalkyl-, Hydroxy-, Alkoxy-, Aralkoxy-, Pyridylalkoxy-, Alkylsulfenyl-, Alkylsulfinyl-, Alkylsulfonyl-, Amino-, Alkylamino-, Dialkylamino-, Alkylcarbonylamino-, Arylcarbonylamino-, Alkylsulfonylamino-, Arylsulfonylamino-, Aralkylamino-, Aminocarbonylalkyl-, Alkylaminocarbonylalkyl-, Dialkylaminocarbonylalkyl-, $R_2$-, $R_2$CO-alkyl- oder $R_3$CO-CH$R_4$-$(CH_2)_l$-NHCO-alkyl-Gruppen mono- oder disubstituiert sein können, wobei die Substituenten gleich oder verschieden sein können und zusätzlich in einem der vorstehend erwähnten ein oder zwei Stickstoffatome enthaltenden 6-gliedrigen heteroaromatischen Reste eine oder zwei -N = CH-Gruppen durch eine oder zwei -N$R_5$-CO-Gruppen ersetzt sein können, in denen

1 die Zahl 0 oder 1,

$R_2$ eine Azetidino-, Pyrrolidino-, Piperidino- oder Hexamethyleniminogruppe, wobei die Methylengruppe in 4-Stellung einer Piperidinogruppe zusätzlich durch eine -O-, -S-, -SO-, -$SO_2$-, -NH-, -N(Alkyl)-, -N(CHO)-, -N(COAlkyl)- oder -N(COAryl)-gruppe ersetzt sein kann,

$R_3$ eine Hydroxygruppe oder eine Alkoxygruppe mit 1 bis 5 Kohlenstoffatomen, in der der Alkoxyteil in 1-, 2- oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino- oder Thiomorpholinogruppe substituiert sein kann,

$R_4$ ein Wasserstoffatom, eine geradkettige oder verzweigte Alkylgruppe mit 1 bis 6 Kohlenstoffatomen, die durch eine Hydroxy-, Mercapto-, Alkylmercapto-, Amino-, $R_3$CO-, Aminocarbonyl-, Phenyl-, Indolyl- oder Imidazolylgruppe substituiert sein kann, wobei $R_3$ wie eingangs definiert ist und der Phenylrest durch ein Fluor-, Chlor- oder Bromatom, durch eine Hydroxy- oder Aminogruppe substituiert sein kann, und

$R_5$ ein Wasserstoffatom, eine Alkyl-, Aralkyl-, Aryl-, Pyridylalkyl-, Aminocarbonylalkyl-, Alkylaminocarbonyl alkyl-, Dialkylaminocarbonylalkyl-, $R_2$CO-alkyl- oder $R_3$CO-CH$R_4$-$(CH_2)_l$-NHCO-alkyl-Gruppe, wobei l, $R_2$, $R_3$ und $R_4$ wie vorstehend erwähnt definiert sind, darstellen,

eine Thiophenylen-, Thiazolylen- oder Thiadiazolylengruppe,

$X_3$ die für $X_2$ vorstehend erwähnten Bedeutungen mit der Maßgabe besitzt, daß $X_3$ keine Thiophenylen-, Thiazolylen- oder Thiadiazolylengruppe und mindestens einer der Reste $X_2$ oder $X_3$ einen der vorstehend erwähnten heteroaromatischen Reste darstellt, und $X_3$ eine 1,2,4-Triazinylengruppe, die im Kohlenstoffgerüst durch ein Fluor-, Chlor-, Brom- oder Jodatom, durch eine Alkyl-, Aralkyl-, Aryl-, Pyridylalkyl-, Hydroxy-, Alkoxy-, Aralkoxy-, Pyridylalkoxy-, Alkylsulfenyl-, Alkylsulfinyl-, Alkylsulfonyl-,

Amino-, Alkylamino-, Dialkylamino-, Alkylcarbonylamino-, Arylcarbonylamino-, Alkylsulfonylamino-, Arylsulfonylamino-, Aralkylamino-, Aminocarbonylalkyl-, Alkylaminocarbonylalkyl-, Dialkylaminocarbonylalkyl-, $R_2$-, $R_2$CO-alkyl- oder $R_3$CO-CHR$_4$-($CH_2$)$_l$-NHCO-alkyl-Gruppe substituiert sein kann, wobei l, $R_2$, $R_3$ und $R_4$ wie vorstehend erwähnt definiert sind und gleichzeitig in einer vorstehend erwähnten 1,2,4-Triazinylengruppe eine oder zwei -N = CH-Gruppen durch eine oder zwei -NR$_5$-CO-Gruppe ersetzt sein können, wobei $R_5$ wie vorstehend erwähnt definiert ist und zusätzlich in einem bei der Definition des Restes $X_3$ vorstehend erwähnten Ringe, die eine -NR$_5$-CO-Gruppe enthalten, $R_5$ eine Bindung zu dem Rest $X_2$ oder auch, wenn $Y_1$ eine Bindung darstellt, zu dem Rest $Y_2$ bedeuten kann,

$Y_1$ eine Bindung, eine -O-, -S-, -SO-, -$SO_2$-, -CO-, -NR$_6$-, -NR$_6$CO-, -CONR$_6$-, -$SO_2$NR$_6$- oder -NR$_6$SO$_2$-Gruppe, wobei

$R_6$ ein Wasserstoffatom, eine Alkylgruppe mit 1 bis 3 Kohlenstoffatomen oder eine Aralkylgruppe darstellt,

oder auch eine -OCH$_2$CO-Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylen- oder Alkinylengruppe mit jeweils 2 bis 6 Kohlenstoffatomen, wobei die Doppelbindung nicht mit einem Sauerstoff-, Schwefel- oder Phosphoratom der Reste $Y_1$, $Y_3$ oder E und die Dreifachbindung nicht mit einem Heteroatom der Reste $Y_1$, $Y_3$ oder E direkt verknüpft sein kann, eine Cycloalkylengruppe mit 3 bis 7 Kohlenstoffatomen oder eine gegebenenfalls durch Fluor-, Chlor-, Brom- oder Jodatome, durch Alkyl-, Hydroxy-, Alkoxy-, Alkylsulfenyl-, Alkylsulfinyl-, Alkylsulfonyl-, Amino-, Alkylcarbonylamino- oder Alkylsulfonylaminogruppen mono- oder disubstituierte Arylengruppe, wobei die Substituenten gleich oder verschieden sein können,

$Y_3$ eine Bindung, eine -CO- oder -CONR$_6$-Gruppe oder auch, wenn ein Heteroatom des Restes $Y_1$ nicht an dasselbe Kohlenstoffatom des Restes $Y_2$ gebunden ist wie der Rest $Y_3$, eine -O-, -S-, -SO-, -$SO_2$-, -NR$_6$CO- oder -NR$_7$-Gruppe, wobei

$R_7$ ein Wasserstoffatom, eine Alkyl-, Aralkyl-, Formyl-, Alkylcarbonyl-, Arylcarbonyl-, Aralkylcarbonyl-, Alkylsulfonyl-, Arylsulfonyl- oder Aralkylsulfonylgruppe darstellt,

eine Gruppe der Formeln

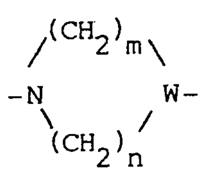

oder

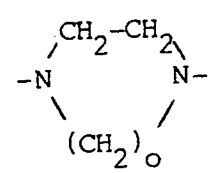

in denen

W eine >CH-, >C(OH)-, >CH-O-, >C = CH- oder >C(CH$_2$COR$_3$)-Gruppe, wobei $R_3$ wie vorstehend erwähnt definiert ist,

m und n jeweils die Zahlen 1, 2 oder 3, wobei jedoch m + n die Zahl 2, 3, 4, 5 oder 6 darstellen muß, und

o die Zahl 2 oder 3 darstellen, wobei in den vorstehend erwähnten Ringen zusätzlich eine Methylengruppe durch eine Carbonylgruppe ersetzt sein kann oder auch, falls die Methylengruppe nicht benachbart zu einem Stickstoffatom steht, durch eine Hydroxygruppe substituiert sein kann, sowie generell ein Sauerstoff- oder Schwefelatom des Restes $Y_3$ nicht unmittelbar auf ein Sauerstoff- oder Schwefelatom oder eine CO-Gruppe des Restes $Y_1$ und ein Sauerstoffatom oder eine Sulfenyl- oder Sulfinylgruppe des Restes $Y_3$ nicht unmittelbar auf ein Stickstoffatom des Restes $Y_1$ und eine CO-Gruppe des Restes $Y_3$ nicht unmittelbar auf eine -O-, -S-, -SO- oder -$SO_2$-Gruppe des Restes $Y_1$ folgen kann,

$Y_4$ eine Bindung, eine geradkettige oder verzweigte Alkylengruppe mit 1 bis 4 Kohlenstoffatomen oder eine gegebenenfalls durch Fluor-, Chlor-, Brom- oder Jodatome, durch Alkyl-, Hydroxy-, Alkoxy-, Alkylsulfenyl-, Alkylsulfinyl-, Alkylsulfonyl-, Amino-, Alkylcarbonylamino- oder Alkylsulfonylaminogruppen mono- oder disubstituierte Arylengruppe, wobei die Substituenten gleich oder verschieden sein können, und

E eine Sulfo-, 5-Tetrazolyl-, Phosphono-, O-Alkyl-phosphono-, Dialkylphosphoryl-, R'-CO-O-(R''CH)-O-CO-, R'''CO- oder R'O-CO-O-(R''CH)-O-CO-Gruppe, wobei

R' und R'' wie vorstehend erwähnt definiert sind und

R''' eine Hydroxygruppe, eine Alkoxygruppe mit 1 bis 5 Kohlenstoffatomen, in der der Alkoxyteil in 1-, 2- oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino- oder Thiomorpholinogruppe substituiert sein kann, eine Arylalkenyloxygruppe mit 3 oder 4 Kohlenstoffatomen im Alkenylteil, eine Cycloalkoxy- oder Cycloalkylalkoxygruppe darstellt,

bedeuten, und mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,

wobei, soweit nichts anderes erwähnt wurde,

unter "eine Aryl- oder Arylengruppe" eine gegebenenfalls durch Fluor-, Chlor-, Brom- oder Jodatome, durch Alkyl-, Trifluormethyl-, Nitro-, Amino-, Alkylamino-, Dialkylamino-, Alkanoylamino-, Alkylsulfonylamino-, Aminosulfonyl-, Alkylaminosulfonyl-, Dialkylaminosulfonyl-, Hydroxy-, Alkoxy-, Carboxy-, Alkoxycarbonyl-, Aminocarbonyl-, Alkylaminocarbonyl-, Dialkylaminocarbonyl-, Alkylsulfenyl-, Alkylsulfinyl- oder Alkylsulfonylgruppen mono-, di- oder trisubstituierte Phenyl- oder Phenylengruppe, wobei die Substituenten gleich oder verschieden sein können, und

die vorstehend erwähnten Alkyl-, Alkylen- oder Alkoxyteile jeweils 1 bis 3 Kohlenstoffatome, die Cycloalkyl- und Cycloalkoxyteile jeweils 3 bis 7 Kohlenstoffatome und die Alkanoylteile 1 bis 4 Kohlenstoffatome enthalten können,

deren Tautomere, deren Stereoisomere, deren Gemische und deren Salze.

**2.** Heterobiarylderivate der allgemeinen Formel I gemäß Anspruch 1, in der

$R_1$ ein Wasserstoffatom, eine Alkylgruppe mit 1 bis 4 Kohlenstoffatomen oder eine gegebenenfalls im Alkoxyteil durch eine Phenylgruppe substituierte Alkoxycarbonylgruppe mit insgesamt 2 bis 5 Kohlenstoffatomen, eine Phosphonogruppe, eine O-Alkyl-phosphono- oder Dialkylphosphorylgruppe, in denen der Alkylteil jeweils 1 oder 2 Kohlenstoffatome enthalten kann, oder eine R'-CO-O-(R''CH)-O-CO-Gruppe, wobei

R' eine Alkylgruppe mit 1 bis 4 Kohlenstoffatomen oder eine Cycloalkylgruppe mit 5 oder 6 Kohlenstoffatomen und

R'' ein Wasserstoffatom oder eine Methylgruppe darstellen,

$X_1$ eine Alkylengruppe mit 1 oder 2 Kohlenstoffatomen oder eine -C(=NH)-Gruppe,

$X_2$ eine Phenylen-, Pyridinylen-, Pyrazinylen-, Pyrimidinylen- oder Pyridazinylengruppe, die jeweils im Kohlenstoffgerüst durch ein Fluor-, Chlor-, Brom- oder Jodatom, durch eine Alkyl-, Amino-, Hydroxy-, Alkoxy-, Pyrrolidino-, Piperidino-, Morpholino-, Thiomorpholino- oder N-Acetylpiperazinogruppe substituiert sein können und in denen der Alkyl- oder Alkoxyteil jeweils 1 oder 2 Kohlenstoffatome enthalten kann, oder eine Thiazolylengruppe,

$X_3$ die für $X_2$ vorstehend erwähnten Bedeutungen mit der Maßgabe besitzt, daß $X_3$ keine Thiazolylengruppe und mindestens einer der Reste $X_2$ oder $X_3$ einen der vorstehend erwähnten heteroaromatischen Reste darstellt, und $X_3$ eine 1,2,4-Triazinylengruppe, wobei in den bei der Definition des Restes $X_3$ vorstehend erwähnten heteroaromatischen Ringen gleichzeitig eine oder zwei -N=CH-Gruppen durch eine oder zwei $-NR_5$-CO-Gruppen ersetzt sein können, in der

$R_5$ ein Wasserstoffatom, eine Alkyl-, Phenyl-, Benzyl-, Aminocarbonylalkyl-, Alkylaminocarbonylalkyl-, Dialkylaminocarbonylalkyl-, $R_2$CO-alkyl- oder $R_3$CO-$CHR_4$-NHCO-alkyl-Gruppe darstellt, in denen der Alkylteil jeweils 1 bis 3 Kohlenstoffatome enthalten kann, $R_2$ eine Pyrrolidino-, Piperidino- oder Hexamethyleniminogruppe, in welcher die Methylengruppe in 4-Stellung einer Piperidinogruppe zusätzlich durch eine -O-, -S-, -NH-, -N(Methyl)-, -NCHO- oder $-N(COCH_3)$-Gruppe ersetzt sein kann, und $R_5$ zusätzlich in einem bei der Definition des Restes $X_3$ vorstehend erwähnten Ringe, die eine $-NR_5$-CO-Gruppe enthalten, eine Bindung zu dem Rest $X_2$ oder auch, wenn $Y_1$ eine Bindung darstellt, zu dem Rest $Y_2$ bedeuten kann,

$R_3$ eine Hydroxygruppe oder eine Alkoxygruppe mit 1 bis 5 Kohlenstoffatomen, in der der Alkoxyteil in 1- oder 2-Stellung durch eine Aryl- oder Pyridylgruppe oder in 2-Stellung durch eine Morpholino- oder Thiomorpholinogruppe substituiert sein kann, und

$R_4$ eine Alkylgruppe mit 1 bis 4 Kohlenstoffatomen, die in 1- oder 2-Stellung durch eine Phenyl- oder $R_3$CO-Gruppe substituiert sein kann, wobei $R_3$ wie eingangs definiert ist und der Phenylrest durch ein Chlor- oder Bromatom, durch eine Hydroxy- oder Aminogruppe substituiert sein kann, darstellen,

$Y_1$ eine Bindung, eine -O-, -CO-, -NH-, $-NCH_3$-, -CONH-, $-CONCH_3$- oder $-SO_2$NH-Gruppe oder auch eine $-OCH_2$CO-Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylengruppe mit 2 bis 6 Kohlenstoffatomen, wobei die Doppelbindung

nicht mit einem Sauerstoff-, Schwefel- oder Phosphoratom der Reste $Y_1$, $Y_3$ oder E direkt verknüpft sein kann, eine Cyclohexylengruppe oder eine gegebenenfalls durch ein Fluor-, Chlor- oder Bromatom oder durch eine Methylgruppe substituierte Phenylengruppe,
$Y_3$ eine Bindung, eine -CO-, -CONH- oder -$CONCH_3$-Gruppe oder auch, wenn ein Heteroatom des Restes $Y_1$ nicht an dasselbe Kohlenstoffatom des Restes $Y_2$ gebunden ist wie der Rest $Y_3$, eine -O-, -NH-, -N($COCH_3$)-, -N(Benzoyl)- oder -N($SO_2CH_3$)-Gruppe,
eine Gruppe der Formeln

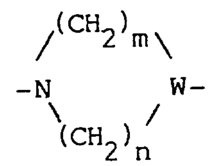

oder

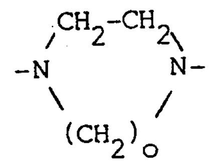

in denen
W eine >CH-, >C(OH)-, >CH-O-, >C=CH- oder >C($CH_2COR_3$)-Gruppe, wobei $R_3$ wie vorstehend erwähnt definiert ist,
m und n jeweils die Zahlen 1, 2 oder 3, wobei jedoch m + n die Zahl 3 oder 4 darstellen muß, und
o die Zahl 2 bedeuten, wobei in den vorstehend erwähnten Ringen zusätzlich eine Methylengruppe durch eine Carbonylgruppe ersetzt sein kann oder auch, falls die Methylengruppe nicht benachbart zu einem Stickstoffatom steht, durch eine Hydroxygruppe substituiert sein kann, sowie generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,
$Y_4$ eine Bindung, eine geradkettige oder verzweigte Alkylengruppe mit 1 bis 4 Kohlenstoffatomen oder eine gegebenenfalls durch ein Fluor-, Chlor- oder Bromatom oder durch eine Methylgruppe substituierte Phenylengruppe und
E eine Sulfo-, 5-Tetrazolyl-, Phosphono-, O-Methyl-phosphono-, R'-CO-O-(R"CH)-O-CO-, R'''CO- oder R'O-CO-O-(R"CH)-O-CO-Gruppe, wobei
R' und R" wie vorstehend erwähnt definiert sind und
R''' eine Hydroxygruppe, eine Alkoxygruppe mit 1 bis 5 Kohlenstoffatomen, in der der Alkoxyteil in 1- oder 2-Stellung durch eine Phenyl- oder Pyridylgruppe oder in 2- oder 3-Stellung durch eine Pyrrolidino-, Piperidino-, Hexamethylenimino-, Morpholino- oder Thiomorpholinogruppe substituiert sein kann, eine Cycloalkoxygruppe mit 4 bis 7 Kohlenstoffatomen, eine Cycloalkylalkoxygruppe mit 4 bis 7 Kohlenstoffatomen im Cycloalkylteil und 1 bis 3 Kohlenstoffatomen im Alkoxyteil, oder eine Phenylallyloxygruppe darstellen,
bedeuten, und mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,
sowie deren Tautomere, deren Stereoisomere, deren Gemische und deren Salze.

**3.** Heterobiarylderivate der allgemeinen Formel I gemäß Anspruch 1, in der
$R_1$ ein Wasserstoffatom, eine Alkylgruppe mit 1 bis 4 Kohlenstoffatomen, eine Alkoxycarbonylgruppe mit insgesamt 2 bis 5 Kohlenstoffatomen, eine Benzyloxycarbonyl-, Dimethylphosphoryl-, Diethylphosphoryl- oder R'-CO-O-(R"CH)-O-CO-Gruppe, wobei
R' eine Methyl- oder Ethylgruppe und
R" ein Wasserstoffatom oder eine Methylgruppe darstellen,
$X_1$ eine Methylengruppe oder eine -C(=NH)-Gruppe,
$X_2$ eine Phenylen-, Pyridinylen-, Pyrazinylen-, Pyrimidinylen- oder Pyridazinylengruppe, die jeweils im Kohlenstoffgerüst durch ein Fluor-, Chlor- oder Bromatom oder durch eine Methyl-, Hydroxy-, Methoxy-, Ethoxy-, Amino-, Dimethylamino-, Pyrrolidino-, Piperidino-, Morpholino-, Thiomorpholino- oder N-Acetylpiperazinogruppe substituiert sein können, oder eine Thiazolylengruppe,
$X_3$ die für $X_2$ vorstehend erwähnten Bedeutungen mit der Maßgabe besitzt, daß $X_3$ keine Thiazolylengruppe und mindestens einer der Reste $X_2$ oder $X_3$ einen der vorstehend erwähnten heteroaromatischen Reste darstellt, und $X_3$ eine 1,2,4-Triazinylengruppe, wobei in den bei der Definition des Restes $X_3$ vorstehend erwähnten heteroaromatischen Ringen gleichzeitig eine -N=CH-Gruppe durch eine -$NR_5$-CO-Gruppe ersetzt sein kann, in der

$R_5$ ein Wasserstoffatom, eine Methyl-, Ethyl-, Phenyl-, Benzyl-, Aminocarbonylmethyl-, Dimethylaminocarbonylmethyl-, $R_2$CO-methyl- oder $R_3$CO-CH$R_4$-NHCO-methyl-Gruppe darstellt, wobei $R_2$ eine Morpholinogruppe,

$R_3$ eine Hydroxygruppe oder eine Alkoxygruppe mit 1 bis 3 Kohlenstoffatomen und

$R_4$ eine Methyl- oder Benzylgruppe und $R_5$ zusätzlich in einem bei der Definition des Restes $X_3$ vorstehend erwähnten Ringe, die eine -N$R_5$-CO-Gruppe enthalten, eine Bindung zu dem Rest $X_2$ oder auch, wenn $Y_1$ eine Bindung darstellt, zu dem Rest $Y_2$ bedeuten kann, darstellen,

$Y_1$ eine Bindung, eine -O-, -CO-, -NH-, -$NCH_3$-, -CONH-, -$CONCH_3$- oder -$SO_2$NH-Gruppe oder auch eine -$OCH_2$CO-Gruppe, wenn $Y_2$ eine Bindung und $Y_3$ eine Piperidinylengruppe darstellen,

$Y_2$ eine Bindung, eine geradkettige oder verzweigte Alkylengruppe mit 1 bis 6 Kohlenstoffatomen, eine geradkettige oder verzweigte Alkenylengruppe mit 2 bis 6 Kohlenstoffatomen, wobei die Doppelbindung nicht mit einem Sauerstoff-, Schwefel- oder Phosphoratom der Reste $Y_1$, $Y_3$ oder E direkt verknüpft sein kann, eine Cyclohexylen- oder Phenylengruppe,

$Y_3$ eine Bindung, eine -CO-, -CONH- oder -$CONCH_3$-Gruppe oder auch, wenn ein Heteroatom des Restes $Y_1$ nicht an dasselbe Kohlenstoffatom des Restes $Y_2$ gebunden ist wie der Rest $Y_3$, eine -O-, -NH-, -N($COCH_3$)-, -N(Benzoyl)- oder -N($SO_2CH_3$)-Gruppe,

eine Gruppe der Formeln

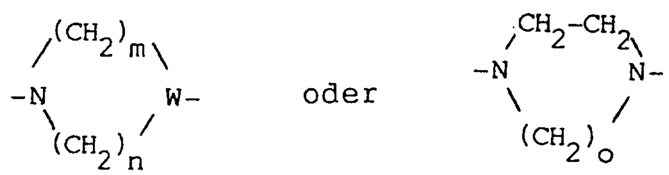

in denen

W eine >CH-, >C(OH)-, >CH-O-, >C=CH- oder >C($CH_2COR_3$)-Gruppe, wobei $R_3$ wie vorstehend erwähnt definiert ist,

m und n jeweils die Zahlen 1, 2 oder 3, wobei jedoch m + n die Zahl 3 oder 4 darstellen muß, und

o die Zahl 2 bedeuten, wobei in den vorstehend erwähnten Ringen zusätzlich eine Methylengruppe durch eine Carbonylgruppe ersetzt sein kann sowie generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,

$Y_4$ eine Bindung, eine geradkettige oder verzweigte Alkylengruppe mit 1 bis 4 Kohlenstoffatomen oder eine Phenylengruppe und

E eine Sulfo-, 5-Tetrazolyl-, Phosphono-, O-Methyl-phosphono- oder R'''CO-Gruppe, wobei

R''' eine Hydroxygruppe, eine Alkoxygruppe mit 1 bis 4 Kohlenstoffatomen, eine Cycloalkoxy- oder Cycloalkoxymethoxygruppe mit jeweils 5 oder 6 Kohlenstoffatomen im Cycloalkoxyteil, eine Benzyloxy- oder Pyridylmethoxygruppe darstellt,

bedeuten, und mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,

sowie deren Tautomere, deren Stereoisomere, deren Gemische und deren Salze.

**4.** Heterobiarylderivate der allgemeinen Formel I gemäß Anspruch 1, in der

$R_1$ ein Wasserstoffatom oder eine Alkoxycarbonylgruppe mit insgesamt 2 oder 3 Kohlenstoffatomen,

$X_1$ eine Methylengruppe oder eine -C(=NH)-Gruppe,

$X_2$ eine Phenylengruppe,

$X_3$ eine Pyrimidinylen- oder Pyridazinylengruppe, die jeweils im Kohlenstoffgerüst durch eine Methoxy- oder Morpholinogruppe substituiert sein können, wobei in den vorstehend erwähnten heteroaromatischen Ringen gleichzeitig eine -N=CH-Gruppe durch eine -N$R_5$-CO-Gruppe ersetzt sein kann, wobei

$R_5$ ein Wasserstoffatom, eine Methyl-, Benzyl- oder Morpholinocarbonylmethylgruppe darstellt,

$Y_1$ eine Bindung, eine -O-, -CO-, -NH-, -$NCH_3$-, -CONH- oder -$CONCH_3$-Gruppe,

$Y_2$ eine Bindung, eine geradkettige oder verzweigte Alkylengruppe mit 1 bis 4 Kohlenstoffatomen, eine Cyclohexylen- oder Phenylengruppe,

$Y_3$ eine Bindung, eine -O-Gruppe oder eine Gruppe der Formel

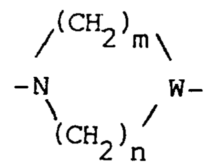

in der
W eine >CH- oder >CH-O-Gruppe,
m und n jeweils die Zahlen 1, 2 oder 3, wobei jedoch m + n die Zahl 4 darstellen muß, bedeuten, sowie generell ein Sauerstoffatom des Restes $Y_3$ nicht unmittelbar auf ein Sauerstoffatom oder eine CO-Gruppe des Restes $Y_1$ und ein Sauerstoffatom des Restes $Y_3$ nicht unmittelbar auf ein Stickstoffatom des Restes $Y_1$ und eine CO-Gruppe des Restes $Y_3$ nicht unmittelbar auf eine -O-Gruppe des Restes $Y_1$ folgen kann,
$Y_4$ eine Bindung, eine Alkylengruppe mit 1 oder 2 Kohlenstoffatomen oder eine Phenylengruppe und
E eine Carboxygruppe oder eine Alkoxycarbonylgruppe mit insgesamt 2 bis 4 Kohlenstoffatomen bedeuten, wobei mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,
sowie deren Tautomere, deren Stereoisomere, deren Gemische und deren Salze.

**5.** Heterobiarylderivate der allgemeinen Formel I gemäß Anspruch 1, in der
$R_1$ ein Wasserstoffatom oder eine Alkoxycarbonylgruppe mit insgesamt 2 oder 3 Kohlenstoffatomen,
$X_1$ eine Methylengruppe oder eine -C(=NH)-Gruppe,
$X_2$ eine Phenylengruppe,
$X_3$ eine gegebenenfalls durch eine Methoxygruppe substituierte Pyrimidinylengruppe, eine gegebenenfalls durch eine Methoxy- oder Morpholinogruppe substituierte Pyridazinylengruppe, eine Pyrimidinylen- oder Pyridazinylengruppe, in denen eine -N=CH-Gruppe durch eine -$NR_5$-CO-Gruppe ersetzt ist, wobei
$R_5$ ein Wasserstoffatom, eine Methyl-, Benzyl- oder Morpholinocarbonylmethylgruppe darstellt,
$Y_1$ eine Bindung, eine -CO-, -CONH- oder -$CONCH_3$-Gruppe,
$Y_2$ eine Bindung, eine geradkettige Alkylengruppe mit 3 oder 4 Kohlenstoffatomen oder eine Cyclohexylengruppe,
$Y_3$ eine Bindung oder eine Gruppe der Formel

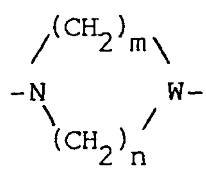

in der
W eine >CH-Gruppe,
m und n jeweils die Zahlen 1, 2 oder 3, wobei jedoch m + n die Zahl 4 darstellen muß, bedeuten,
$Y_4$ eine Bindung oder eine Methylengruppe und
E eine Carboxygruppe oder eine Alkoxycarbonylgruppe mit insgesamt 2 oder 3 Kohlenstoff atomen bedeuten, wobei mindestens einer der Reste $Y_1$, $Y_2$, $Y_3$ oder $Y_4$ keine Bindung darstellt und der Rest E nicht unmittelbar auf ein Heteroatom der Reste $Y_1$ oder $Y_3$ folgen kann,
sowie deren Tautomere, deren Stereoisomere, deren Gemische und deren Salze.

**6.** Folgende Verbindungen der allgemeinen Formel I gemäß Anspruch 1:
(a) 6-(4-Amidinophenyl)-4-[(trans-4-carboxycyclohexyl)-aminocarbonyl]-2-methyl-(2H)-pyridazin-3-on,
(b) 6-(4-Amidinophenyl)43-[[trans-4-(methoxycarbonyl)-cyclohexyl]-aminocarbonyl]-2-methyl-(2H)-pyridazin-3-on,
(c) 6-[4-[N-(Methoxycarbonyl)-amidino]-phenyl]-4-[[trans-4-(methoxycarbonyl)-cyclohexyl]-aminocarbonyl]-2-methyl-(2H)-pyridazin-3-on,
(d) 6-[4-[N-(Ethoxycarbonyl)-amidino]-phenyl]-4-[[trans-4-(methoxycarbonyl)-cyclohexyl]-aminocarbonyl]-2-methyl-(2H)-pyridazin-3-on,

(e) 2-(4-Amidinophenyl)-5-[4-(carboxymethyl)-piperidinocarbonyl]-4-methoxy-pyrimidin,
(f) 2-(4-Amidinophenyl)-4-methoxy-5-[4-[(methoxycarbonyl)-methyl]-piperidinocarbonyl]-pyrimidin,
(g) 4-Methoxy-2-[4-[N-(methoxycarbonyl)-amidino]-phenyl]-5-[4-[(methoxycarbonyl)-methyl]-piperidinocarbonyl]-pyrimidin,
(h) 6-(4-Amidinophenyl)-4-[N-(trans-4-carboxycyclohexyl)-N-methyl-aminocarbonyl]-2-methyl-(2H)-pyridazin-3-on,
(i) 6-(4-Amidinophenyl)-4-[N-[trans-4-(methoxycarbonyl)-cyclohexyl]-N-methyl-aminocarbonyl]-2-methyl-(2H)-pyridazin-3-on,
(j) 3-(4-Amidinophenyl)-5-[(trans-4-carboxycyclohexyl)-aminocarbonyl]-pyridazin,
(k) 3-(4-Amidinophenyl)-5-[[trans-4-(methoxycarbonyl)-cyclohexyl]-aminocarbonyl]-pyridazin,
(l) 6-(4-Amidinophenyl)-4-[(trans-4-carboxycyclohexyl)-aminocarbonyl]-2-[(morpholinocarbonyl)-methyl]-(2H)-pyridazin-3-on und
(m) 6-(4-Amidinophenyl)-4-[[trans-4-(methoxycarbonyl)-cyclohexyl]-aminocarbonyl]-2-[-(morpholinocarbonyl)-methyl]-(2H)-pyridazin-3-on,

deren Tautomere und deren Salze.

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der Heterobiaryle gemäß den Ansprüchen 1 bis 7, dadurch gekennzeichnet, daß

a) zur Herstellung von Verbindungen der allgemeinen Formel I, in der E eine Carboxygruppe darstellt, eine Verbindung der allgemeinen Formel

$$R_1NH - X_1 - X_2 - X_3 - Y_1 - Y_2 - Y_3 - Y_4 - E' \quad ,(II)$$

in der
$R_1$, $X_1$ bis $X_3$ und $Y_1$ bis $Y_4$ wie in den Ansprüchen 1 bis 6 definiert sind und
E', das an ein Kohlenstoffatom gebunden ist, eine mittels Hydrolyse, Behandlung mit Säuren, Thermolyse oder Hydrogenolyse in eine Carboxygruppe überführbare Gruppe darstellt, in eine entsprechende Carboxyverbindung übergeführt wird oder

b) zur Herstellung von Verbindungen der allgemeinen Formel I, in der die $R_1NH-X_1$-Gruppe eine Amidinogruppe darstellt, in der $R_1$ ein Wasserstoffatom, eine Hydroxy-, Alkyl- oder Aminogruppe bedeutet, eine gegebenenfalls im Reaktionsgemisch gebildete Verbindung der allgemeinen Formel

$$Z_1 - C(=NH) - X_2 - X_3 - Y_1 - Y_2 - Y_3 - Y_4 - E \quad ,(III)$$

in der
$X_2$, $X_3$, $Y_1$ bis $Y_4$ und E wie in den Ansprüchen 1 bis 6 definiert sind und
$Z_1$ eine Alkoxy-, Aralkoxy-, Alkylthio-, Aralkylthio- oder Aminogruppe darstellt, mit einem Amin der allgemeinen Formel

$$R_a - NH_2 \quad ,(IV)$$

in der
$R_a$ ein Wasserstoffatom, eine Hydroxy-, Alkyl- oder Aminogruppe bedeutet, oder mit deren Säureadditionssalzen umgesetzt wird oder
c) zur Herstellung von Verbindungen der allgemeinen Formel I, in der die $R_1NH-X_1$-Gruppe eine Aminoalkylgruppe darstellt, eine Verbindung der allgemeinen Formel

$NC - X_1' - X_2 - X_3 - Y_1 - Y_2 - Y_3 - Y_4 - E$ ,(V)

in der
$X_2$, $X_3$, $Y_1$ bis $Y_4$ und E wie in den Ansprüchen 1 bis 6 definiert sind und
$X_1'$ eine Bindung oder eine Alkylengruppe mit 1 oder 2 Kohlenstoffatomen darstellt, reduziert wird oder
d) zur Herstellung von Verbindungen der allgemeinen Formel I, in der $R_1$ eine Alkoxycarbonyl-, Aralkoxycarbonyl-, Aryloxycarbonyl-, Alkylcarbonyl- oder Arylcarbonylgruppe, in denen der Alkyl- und Alkoxyteil jeweils 1 bis 4 Kohlenstoffatome enthalten kann, eine Alkenyloxycarbonylgruppe mit insgesamt 4 bis 6 Kohlenstoffatomen, eine Phosphono-, O-Alkylphosphono-, Dialkylphosphoryl- oder R'-CO-O-(R''CH)-O-CO-Gruppe darstellt, wobei R' und R'' wie in den Ansprüchen 1 bis 6 definiert sind, eine Verbindung der allgemeinen Formel

$H_2N - X_1 - X_2 - X_3 - Y_1 - Y_2 - Y_3 - Y_4 - E$ ,(VI)

in der
$X_1$ bis $X_3$, $Y_1$ bis $Y_4$ und E wie in den Ansprüchen 1 bis 6 definiert sind, mit einer Verbindung der allgemeinen Formel

$Z_2 - R_b$ ,(VII)

in der
$R_b$ eine Alkoxycarbonyl-, Aralkoxycarbonyl-, Aryloxycarbonyl-, Alkylcarbonyl- oder Arylcarbonylgruppe, in denen der Alkyl- und Alkoxyteil jeweils 1 bis 4 Kohlenstoffatome enthalten kann, eine Alkenyloxycarbonylgruppe mit insgesamt 4 bis 6 Kohlenstoffatomen, eine R'-CO-O-(R''CH)-O-CO- oder Dialkylphosphorylgruppe darstellt, wobei R' und R'' wie in den Ansprüchen 1 bis 6 definiert sind, und
$Z_2$ eine nukleophile Austrittsgruppe darstellen, umgesetzt und gegebenenfalls anschließend ein oder zwei Alkylreste von einer so erhaltenen Dialkylphosphorylverbindung abgespalten werden oder
e) zur Herstellung von Verbindungen der allgemeinen Formel I, in der E eine R'''CO-Gruppe darstellt, wobei R''' wie in den Ansprüchen 1 bis 6 definiert ist, eine Verbindung der allgemeinen Formel

$R_1NH - X_1 - X_2 - X_3 - Y_1 - Y_2 - Y_3 - Y_4 - E''$ ,(VIII)

in der
$R_1$, $X_1$ bis $X_3$ und $Y_1$ bis $Y_4$ wie in den Ansprüchen 1 bis 6 definiert sind und
E'' eine Carboxy- oder Alkoxycarbonylgruppe darstellt, mit einem Alkohol der allgemeinen Formel

HO - R''' ,(IX)

in der
R''' wie in den Ansprüchen 1 bis 6 definiert ist, umgesetzt wird oder
f) zur Herstellung von Verbindungen der allgemeinen Formel I, in der $Y_1$ eine -CO- oder $-CONR_6$-Gruppe darstellt, eine Verbindung der allgemeinen Formel

$R_1NH - X_1 - X_2 - X_3 -COOH$ ,(X)

in der
$R_1$ und $X_1$ bis $X_3$ wie in den Ansprüchen 1 bis 6 definiert sind, mit einer Verbindung der allgemeinen Formel

$U - Y_2 - Y_3 - Y_4 - E$ ,(XI)

in der
$Y_2$ bis $Y_4$ und E wie in den Ansprüchen 1 bis 6 definiert sind und
U, falls $Y_2$ keine Bindung darstellt, eine $-NR_7$-Gruppe, wobei $R_7$ wie in den Ansprüchen 1 bis 6 definiert ist, oder, wenn $Y_3$ eine der in den Ansprüchen 1 bis 6 erwähnten cyclischen Iminogruppen und $Y_2$ eine Bindung darstellen, auch ein Wasserstoffatom bedeuten, oder mit deren reaktionsfähigen Derivaten umgesetzt wird oder
g) zur Herstellung der Verbindungen der allgemeinen Formel I, in der E eine R'-CO-O-(R''CH)-O-CO-, R'O-CO-O-(R''CH)-O-CO-oder R'''CO-Gruppe darstellt, wobei R' bis R''' wie in den Ansprüchen 1 bis 6 definiert sind, eine Verbindung der allgemeinen Formel

$R_1NH - X_1 - X_2 - X_3 - Y_1 - Y_2 - Y_3 - Y_4 - COOH$ ,(XII)

in der
$R_1$, $X_2$ bis $X_3$ und $Y_1$ bis $Y_4$ wie in den Ansprüchen 1 bis 6 definiert sind, mit einer Verbindung der allgemeinen Formel

$Z_3 - R_c$ ,(XIII)

in der
$R_c$ eine R'-CO-O-(R''CH)-, R'O-CO-O-(R''CH)- oder R'''-Gruppe, wobei R' bis R''' wie in den Ansprüchen 1 bis 6 definiert sind, und
$Z_3$ eine nukleophile Austrittsgruppe darstellen, umgesetzt wird und
erforderlichenfalls anschließend ein während der Umsetzungen zum Schutze von reaktiven Gruppen verwendeter Schutzrest abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 7507
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| A | GB-A-2 095 240 (FUJISAWA PHARMACEUTICAL)<br>* Seite 9; Ansprüche *<br>--- | 1,7-9 | C07D237/24<br>C07D239/36<br>C07D213/64<br>C07D237/14<br>C07D237/20<br>C07D401/06<br>C07F9/6512<br>A61K31/50 |
| A | EP-A-0 085 985 (MITSUBISHI CHEMICAL INDUSTRIES)<br>* Seite 7; Ansprüche *<br>--- | 1,6-9 | |
| A | EP-A-0 351 856 (TAKEDA CHEMICAL INDUSTRIES)<br>* Seite 67 - Seite 70; Ansprüche *<br>--- | 1,6-9 | |
| A | CHEMICAL ABSTRACTS, vol. 103, no. 28, 1985, Columbus, Ohio, US;<br>abstract no. 87902n,<br>Seite 613 ;Spalte 1 ;<br>* Zusammenfassung * | 1,6 | |
| A | & JP-A-6 025 973 (CHISSO CORP.)<br>8. Februar 1985<br>---<br>-/-- | 1,6 | |

RECHERCHIERTE SACHGEBIETE (Int. Cl. 5)

C07D
C07F
A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 14 JANUAR 1993 | FRANCOIS J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER TEILRECHERCHENBERICHT

Nummer der Anmeldung

EP 92 11 7507
Seite 2

| EINSCHLÄGIGE DOKUMENTE | | | KLASSIFIKATION DER ANMELDUNG (Int. Cl. 5) |
|---|---|---|---|
| Kategorie | Kennzeichnung des Dokuments mit Angabe, soweit erforderlich der maßgeblichen Teile | Betrifft Anspruch | |
| A | JOURNAL OF MEDICINAL CHEMISTRY<br>Bd. 23, 1980, WASHINGTON US<br>Seiten 578 - 581<br>B. DAS ET AL. 'SYNTHESIS AND ANTIPYRANOSOMAL ACTIVITY OF SOME BIS(4-GUANYLPHENYL)FIVE AND SIX-MEMBERED RING HETEROCYCLES.'<br>* Seite 578 - Seite 579; Tabelle 1 *<br>--- | 1,6-9 | |
| A | PHARMAZIE<br>Bd. 35, Nr. 5-6, 1980, BERLIN DD<br>Seiten 285 - 288<br>G. WAGNER ET AL. 'SYNTHESE VON 2-(4-AMIDINOPHENYL)BENZOFURAN-,BENZOXAZOL- UND -BENZOTHIAZOL SOWIE VON 2(4-AMIDINOBENZYL)BENZOXAZOL'<br>* Seite 285 - Seite 287 *<br>----- | 1,7-9 | RECHERCHIERTE SACHGEBIETE (Int. Cl. 5) |

EP 92 11 7507 -C-

Vollständig recherchierte Patentansprüche : 6-11
Unvollständig recherchierte Patentansprüche : 1-5

Grund : Die Zahl, der im Patentansprüche 1 bis 5 beschriebene Heterobiaryldervate, ist so gross das eine vollständige Recherche auf ökonomischen Grunde nicht möglich ist (Richtlinien EPO, Teil B, III, 2).

Die Recherche beschränkt sich deshalb auf die Beispielsubstanzen (Rule 45).